# EUROPEAN PATENT SPECIFICATION

(11) **EP 2 240 101 B1**
(45) Date of publication and mention of the grant of the patent: **17.08.2011**
(21) Application number: 09726616.7
(22) Date of filing: 31.03.2009
(51) Int. Cl.: A61B 17/70, A61B 17/88

(54) **HYBRID DYNAMIC STABILIZATION**
HYBRID DYNAMISCHE STABILISIERUNG
STABILISATION DYNAMIQUE HYBRIDE

(30) Priority: 31.03.2008 US 59634
(43) Date of publication of application: 20.10.2010
(73) Proprietor: Zimmer Spine, Inc., Minneapolis, MN 55439-2027 (US)
(72) Inventor: JOHNS, Derrick, William, Austin TX 78717 (US)
(74) Representative: Manitz, Finsterwald & Partner GbR
(86) International application number: PCT/US2009/038977
(87) International publication number: WO 2009/124062

(56) References cited:
- EP-A- 1 815 812
- FR-A- 2 867 057
- FR-A- 2 890 850
- US-A1- 2005 085 815

## Description

### TECHNICAL FIELD OF THE DISCLOSURE

This disclosure relates generally to spinal implants, and more particularly to prophylactic methods and systems relating to adjacent level disease.

### BACKGROUND OF THE DISCLOSURE

Degenerative disc disease (DDD) refers to a syndrome in which a disc in the spine has been compromised. Surgical treatment of lumbar degenerative disc disease (DDD) has evolved along 2 pathways: fusion and arthroplasty. Patients with one-level DDD who have failed non-operative treatment and have intractable low back pain are generally indicated for a fusion procedure.

Modem spine surgery often involves spinal fixation through the use of spinal implants or fixation systems to correct or treat various spine disorders or to support the spine. Spinal implants may help, for example, to stabilize the spine, correct deformities of the spine, facilitate fusion, or treat spinal fractures. A spinal fixation system typically includes corrective spinal instrumentation that is attached to selected vertebra of the spine by screws and hooks. The corrective spinal instrumentation includes spinal rods or plates that are generally parallel to the patient's back. An example of prior spinal rods is disclosed in EP1 815 812 A1 by Spinelab AG. The corrective spinal instrumentation may also include transverse connecting rods that extend between neighboring spinal rods. Spinal fixation systems are used to correct problems in the cervical, thoracic, and lumbar portions of the spine, and are often installed posterior to the spine on opposite sides of the spinous process and adjacent to the transverse process.

Often, spinal fixation may include rigid (i.e., in a fusion procedure) support for the affected regions of the spine. Such systems limit movement in the affected regions in virtually all directions (for example, in a fused region). More recently, so called dynamic" systems have been introduced wherein the implants allow at least some movement of the affected regions in at least some directions, i.e. flexion, extension, lateral bending, or torsion. In many of these systems, dynamic movement is enabled through the use of flexible rods.

A primary complication of fusion procedures is adjacent-level disease (also known as ALD, ALDDD, and transition syndrome) requiring surgical intervention at an adjacent level. There is some debate as to whether transition syndrome in the lumbar spine is caused by the natural progression of degenerative disease, or by the fusion itself. One symptom of ALD may be increased disc pressures and a change in the range of motion (ROM) at levels adjacent to a fusion. This phenomenon may play a role in increasing the likelihood of transition syndrome in patients with a lumbar fusion. Although rarely reported, transition syndrome occurs at an exceptionally high rate. The re-operation rate for symptomatic lumbar adjacent-level disease may be as high as 20%.

Bone may be subject to degeneration caused by trauma, disease, and/or aging. Degeneration may destabilize bone and affect surrounding structures. For example, destabilization of a spine may result in alteration of a natural spacing between adjacent vertebrae.

### SUMMARY OF THE DISCLOSURE

Embodiments disclosed herein may be useful for preventing or slowing down the effects of adjacent level disease.

In accordance with one embodiment, a system for supporting a spine includes a rod having a rigid portion and a dynamic portion, a first anchor for connecting the rigid portion of the rod to a pedicle of a first vertebra, a second anchor for connecting the rigid portion of the rod to a pedicle of a second vertebra, a conformable ligature comprising a first end and a second end and a loop portion for passing around a portion of an adjacent third vertebra, and a blocking body. The blocking body includes a loop passage, an exit passage, an engagement portion, a closure member for engagement with the blocking body engagement portion, and a compression member having a first surface. The loop portion of the conformable ligature passes through the loop passage and the first and second ends extend from the exit passage. The closure member engages with the blocking body in a manner to allow the first surface of the compression member to contact the conformable ligature to create a friction force between the conformable ligature and the blocking body. The blocking body connects the dynamic portion of the rod to the third vertebra to maintain motion of the adjacent third vertebra relative the first and second vertebrae, wherein the first and second vertebrae are rigidly connected. In some embodiments, the rod comprises a cylindrical body with a dynamic portion having a cannulated interior and an opening extending spirally around a longitudinal axis of the cylindrical body; and a biomaterial coating the cylindrical body in whole or in part and at least partially filling the opening. In some embodiments, the opening has an interlocking pattern. In some embodiments, the interlocking pattern has a shape of a dog bone or puzzle. In some embodiments, the opening has a non-linear path. In some embodiments, the opening has a linear path. In some embodiments, the biomaterial fills the cylindrical body in whole or in part. In some embodiments, the biomaterial is polycarbonate urethane. In some embodiments, the loop portion of the conformable ligature passes around a non-pedicle portion of the third vertebra. In some embodiments, the loop portion of the conformable ligature passes around a lamina of the third vertebra.

A method for treating a spine may include connecting a rigid portion of a rod to a pedicle portion of first and second vertebrae to fuse the first and second vertebrae and connecting a dynamic portion of the rod to a non-pedicle portion of an adjacent vertebra. The rod has a cylindrical body, and the dynamic portion has a cannulated interior and an opening extending spirally around a longitudinal axis of the cylindrical body. The rod also has a biomaterial coating the cylindrical body in whole or in part and at least partially filling the opening. In some embodiments, the blocking body comprises a loop passage, an exit passage, an engagement portion, a closure member, and a compression member having a first surface. In some embodiments, the loop portion of the conformable ligature passes through the loop passage and the first and second ends extend from the exit passage. The closure member engages with the blocking body in a manner to allow the first surface of the compression member contacting the conformable ligature to create a friction force between the conformable ligature and the blocking body. The blocking body connects the dynamic portion of the rod to the adjacent third vertebra to maintain motion of the adjacent third vertebra relative the first and second vertebrae and the first and second vertebrae are rigidly connected. Connecting may include passing a loop portion of a conformable ligature around a portion of the spine, passing the loop portion of the conformable ligature through a blocking body, and engaging the closure member with the blocking body engagement portion. Connecting a rigid portion of a rod to a pedicle portion of a first and second vertebrae to fuse the first and second vertebrae can comprise advancing a first anchor into the pedicle of a first vertebra, advancing a second anchor into the pedicle of a second vertebra, and securing the rod to the first and second anchors. In some embodiments, the method comprises a minimally-invasive surgery. In some embodiments, the method comprises connecting the dynamic portion of the rod to the lamina of the third vertebra.

A minimally-invasive surgical method for the treatment of a spine may include advancing a first anchor in a patient to a first vertebra via an incision, connecting a rigid portion of a rod to the first vertebra, advancing a second anchor in the patient to a second vertebra via an incision, connecting the rigid portion of the rod to the second vertebra to prevent motion of the second vertebra relative to the first vertebra, and connecting a dynamic portion of the rod to a non-pedicle portion of a third vertebra adjacent to the first vertebra to connect the adjacent third vertebra dynamically to the first vertebra and the second vertebra. In some embodiments, the rod has a cylindrical body and the dynamic portion has a cannulated interior and an opening extending spirally around a longitudinal axis of the cylindrical body, and a biomaterial coating the cylindrical body in whole or in part and at least partially filling the opening. Connecting the dynamic portion of the rod to a non-pedicle portion of the third vertebra can comprise advancing a conformable ligature into the body via an incision, passing a loop portion of a conformable ligature around a portion of the spine, passing a loop portion of the conformable ligature through a blocking body, and engaging the closure member with the blocking body engagement portion. In some embodiments, the blocking body comprises a loop passage, an exit passage, an engagement portion, a closure member, and a compression member having a first surface. In some embodiments, the closure member engages with the blocking body in a manner to allow the first surface of the compression member contacting the conformable ligature to create a friction force between the conformable ligature and the blocking body. In some embodiments, the loop portion of the conformable ligature passes through the loop passage and the first and second ends extend from the exit passage. The dynamic portion of the rod can be connected to the adjacent third vertebra before connecting the rigid portion to the first vertebra. One or more of the first anchor, the second anchor, the conformable ligature and the blocking body can be advanced via a single incision. The conformable ligature can be passed around a non-pedicle portion of the third vertebra. The conformable ligature can be passed around the lamina of the third vertebra.

In accordance with one feature of the disclosure, a rod has a dynamic portion having a hollow cylindrical body and an opening extending spirally around a longitudinal axis of the cylindrical body. The opening may be machined, etched, or otherwise cut to a shape. According to one feature of the disclosure, the shape has a non-linear path. The shape has an interlocking pattern. In one embodiment, the shape resembles a dog bone. In one embodiment, the shape resembles a puzzle.

In accordance with one feature of the disclosure, a portion or portions of the rod can be left rigid and uncut for integration with other spinal device(s) such as bone fasteners to facilitate fusion or segmental stability of the spine. In accordance with one feature of the disclosure, a dynamic portion of a spine stabilization rod may be filled and/or coated in whole or in part with a biomaterial such as a polymer to prevent over-extension and reduce wear. In one embodiment, the polymer is polycarbonate urethane. In one embodiment, the opening of the dynamic stabilization rod is at least partially filled with the polymer to enhance rigidity of its cylindrical body.

In accordance with one feature of the disclosure, the cylindrical body and the opening of a dynamic portion of a spine stabilization rod may be filled in whole or in part with the same biomaterial or different biomaterials. In one embodiment, the cylindrical body and opening of a dynamic stabilization rod may be filled in whole or in part with polycarbonate urethane.

In accordance with one feature of the disclosure, a dynamic portion of a spine stabilization rod may be coated in whole or in part with a biomaterial such as a polymer. In one embodiment, the polymer is polycarbonate urethane.

An advantage to using a hybrid stabilization system and method may be a reduction or elimination of micro motion between the threaded member and the bone, which may be referred to as dynesis or a "windshield-wiper" effect. Over time, the effect may result in damaged hardware, a loosening of the threaded member, or may damage the pedicle to a point that rigid attachment to a spinal fixation rod is not possible. By using a hybrid stabilization system, the pedicle is not damaged because the ligature may be attached to a lamina, a transverse process, or some other non-pedicle portion of the vertebra. Furthermore, if the patient needs to have an adjacent level fused at some later date, the surgeon is free to select a location because there is not an existing threaded member.

These, and other, aspects will be better appreciated and understood when considered in conjunction with the following description and the accompanying drawings. The following description, while indicating various embodiments and numerous specific details thereof, is given by way of illustration and not of limitation. Many substitutions, modifications, additions or rearrangements may be made within the scope of the disclosure, and the disclosure includes all such substitutions, modifications, additions or rearrangements.

### BRIEF DESCRIPTION OF THE DRAWINGS

Advantages of the present invention will become apparent to those skilled in the art with the benefit of the following detailed description and upon reference to the accompanying drawings in which:

FIGURE 1 depicts a perspective view of an embodiment of a spinal stabilization system;

FIGURE 2 depicts a perspective view of an embodiment of a bone fastener assembly;

FIGURE 3 depicts a cross-sectional view of an embodiment of a bone fastener assembly;

FIGURE 4 depicts a perspective view of an embodiment of a closure member having an external tool portion;

FIGURE 5 is a partial cutaway perspective view showing an embodiment of a blocking body and a dynamic portion of a rod;

FIGURE 6 is a partial cutaway view of an embodiment of a blocking body and a conformable ligature coupled to a rod;

FIGURE 7 is a partial cutaway perspective view an embodiment of a blocking body and conformable ligature;

FIGURE 8 is a partial cutaway view of an embodiment of a blocking body and conformable ligature coupled to a non-pedicle portion of a vertebra;

FIGURE 9 is a perspective view of an embodiment of a blocking body;

FIGURES 10A, 10B and 10C are vertical section views of configurations of the embodiment of the blocking body shown in FIGURE 9;

FIGURE 11 is a partial cutaway view of an embodiment of a blocking body and conformable ligature coupled to a non-pedicle portion of a vertebra;

FIGURE 12 depicts a perspective view of an example of a blocking body;

FIGURE 13 depicts a cross-sectional side view of an example of a blocking body and conformable ligature;

FIGURE 14 depicts a perspective view of an example of a blocking body and conformable ligature;

FIGURE 15 depicts a sagittal view of an embodiment of a spine stabilization system attached to a portion of a spine, illustrating a method for supporting a spine;

FIGURE 16 depicts a side view of an embodiment of a spine stabilization system attached to a portion of a spine, illustrating a method for supporting a spine;

FIGURE 17 depicts a side view of an example of a tensioning tool for tensioning a ligature in a blocking body;

FIGURE 18 depicts an embodiment of a rod having a dynamic portion and a rigid portion;

FIGURE 19 depict an embodiment of a rod having a dynamic portion and a rigid portion;

FIGURE 20 depicts an embodiment of a rod having a dynamic portion and a rigid portion; and

FIGURE 21 depicts an embodiment of a rod having a dynamic portion and a rigid portion.

While the invention is susceptible to various modifications and alternative forms, specific embodiments thereof are shown by way of example in the drawings and will herein be described in detail. The drawings may not be to scale. It should be understood that the drawings and detailed description thereto are not intended to limit the invention to the particular form disclosed, but on the contrary, the intention is to cover all modifications, equivalents, and alternatives falling within the spirit and scope of the present invention as defined by the appended claims.

### DETAILED DESCRIPTION

The invention and the various features and advantageous details thereof are explained more fully with reference to the non-limiting embodiments that are illustrated in the accompanying drawings and detailed in the following description. Descriptions of well known starting materials, processing techniques, components and equipment are omitted so as not to unnecessarily obscure the disclosure in detail. Skilled artisans should understand, however, that the detailed description and the specific examples, while disclosing preferred embodiments, are given by way of illustration only and not by way of limitation. Various substitutions, modifications, additions or rearrangements within the scope of the underlying inventive concept(s) will become apparent to those skilled in the art after reading this disclosure.

As used herein, the terms "comprises," "comprising," "includes," "including," "has," "having" or any other variation thereof, are intended to cover a non-exclusive inclusion. For example, a process, product, article, or apparatus that comprises a list of elements is not necessarily limited to only those elements but may include other elements not expressly listed or inherent to such process, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Additionally, any examples or illustrations given herein are not to be regarded in any way as restrictions on, limits to, or express definitions of, any term or terms with which they are utilized. Instead these examples or illustrations are to be regarded as being described with respect to one particular embodiment and as illustrative only. Those of ordinary skill in the art will appreciate that any term or terms with which these examples or illustrations are utilized encompass other embodiments as well as implementations and adaptations thereof which may or may not be given therewith or elsewhere in the specification and all such embodiments are intended to be included within the scope of that term or terms. Language designating such nonlimiting examples and illustrations includes, but is not limited to: "for example," "for instance," "e.g.," "in one embodiment," and the like.

Reference is now made in detail to the exemplary embodiments, examples of which are illustrated in the accompanying drawings. Wherever possible, the same reference numbers will be used throughout the drawings to refer to the same or like parts (elements).

A spinal stabilization system may be installed in a patient to stabilize a portion of a spine. Spinal stabilization may be used, but is not limited to use, in patients having degenerative disc disease, adjacent level disease, spinal stenosis, spondylolisthesis, pseudoarthrosis, and/or spinal deformities; in patients having fracture or other vertebral trauma; and in patients after tumor resection. A spinal stabilization system may be installed using a minimally invasive procedure. An instrumentation set may include instruments and spinal stabilization system components for forming a spinal stabilization system in a patient.

A minimally invasive procedure may be used to limit an amount of trauma to soft tissue surrounding vertebrae that are to be stabilized. In some embodiments, the natural flexibility of skin and soft tissue may be used to limit the length and/or depth of an incision or incisions needed during the stabilization procedure. Minimally invasive procedures may provide limited direct visibility *in vivo.* Forming a spinal stabilization system using a minimally invasive procedure may include using tools to position system components in the body.

A minimally invasive procedure may be performed after installation of one or more spinal implants in a patient. The spinal implant or spinal implants may be inserted using an anterior procedure and/or a lateral procedure. The patient may be turned and a minimally invasive procedure may be used to install a posterior spinal stabilization system. A minimally invasive procedure for stabilizing the spine may be performed without prior insertion of one or more spinal implants in some patients. In some patients, a minimally invasive procedure may be used to install a spinal stabilization system after one or more spinal implants are inserted using a posterior spinal approach.

A spinal stabilization system may be used to stabilize a spine while minimizing the amount of damage to surrounding tissue. In some embodiments, a spinal stabilization system may be used to stabilize two adjacent vertebrae (i.e., one vertebral level) and provide dynamic stabilization to a third adjacent vertebra. A spinal stabilization system may include bone fastener assemblies in the first and second vertebrae and include a clamp on the third vertebra. A rod may be coupled and secured to the bone fastener assemblies and the clamp. The rod may have a rigid portion and a dynamic portion. The clamp and a conformable ligature may secure the dynamic portion of the rod to a non-pedicle portion of the third vertebra. The first and second vertebrae may be rigidly fixed to each other, and the second and third vertebrae may be dynamically fixed to each other to allow some movement between them. As used herein, "coupled" components may directly contact each other or may be separated by one or more intervening members.

Minimally invasive procedures may reduce trauma to soft tissue surrounding vertebrae that are to be stabilized. Only a small opening may need to be made in a patient. For example, for a stabilization procedure on one side of the spine, the surgical procedure may be performed through a single incision formed in the skin of the patient. In some embodiments, the incision may be above and substantially between the vertebrae to be stabilized. In some embodiments, the incision may be above and between the vertebrae to be stabilized. In some embodiments, the incision may be above and substantially halfway between the vertebrae to be stabilized. Dilators, a targeting needle, and/or a tissue wedge may be used to provide access to the vertebrae to be stabilized without the need to form an incision with a scalpel through muscle and other tissue between the vertebrae to be stabilized. A minimally invasive procedure may reduce an amount of post-operative pain felt by a patient as compared to invasive spinal stabilization procedures. A minimally invasive procedure may reduce recovery time for the patient as compared to invasive spinal procedures.

Components of spinal stabilization systems may be made of materials including, but not limited to, titanium, titanium alloys, stainless steel, ceramics, and/or polymers. Some components of a spinal stabilization system may be autoclaved and/or chemically sterilized. Components that may not be autoclaved and/or chemically sterilized may be made of sterile materials. Components made of sterile materials may be placed in working relation to other sterile components during assembly of a spinal stabilization system.

Spinal stabilization systems may be used to correct problems in lumbar, thoracic, and/or cervical portions of a spine. Various embodiments of a spinal stabilization system may be used from the C1 vertebra to the sacrum. For example, a spinal stabilization system may be implanted posterior to the spine to maintain distraction between adjacent vertebral bodies in a lumbar portion of the spine. FR 2 890 850, assigned to the assignee of the present application, discloses an example of using a ligature to couple a portion of a spinal stabilization and a portion of a bone.

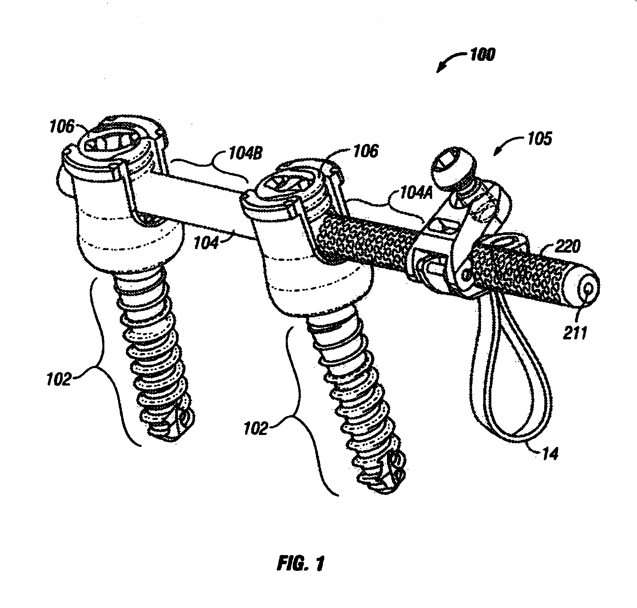

FIGURE 1 depicts an embodiment of spinal stabilization system 100 that may be implanted using a minimally invasive surgical procedure. Spinal stabilization system 100 may include anchors such as bone fastener assemblies 102, rod 104 having a dynamic portion 104A and a rigid portion 104B, clamp 105, conformable ligature 14 and closure members 106. Other spinal stabilization system embodiments may include, but are not limited to, plates, dumbbell-shaped members, and/or transverse connectors. FIGURE 1 depicts a spinal stabilization system for rigid fixation of a first vertebral level and dynamic rigid fixation for an adjacent second vertebral level.

Rod 104 may include dynamic portion 104A and rigid portion 1048. In embodiments disclosed herein, the term "dynamic" refers to the flexing capability of a spinal rod. The flexing capability is configured to provide a bending stiffness or a spring rate that is non-linear with respect to the bending displacement of the rod. This is intended to more closely mimic the ligaments in a normal stable spine which are non-linear in nature. The non-linear bending stiffness of the dynamic stabilization rods disclosed herein is intended to allow the limited initial range of spinal motion and to restrict or prevent spinal motion outside of the limited initial range. In one embodiment, the bending stiffness is produced by configuring the rod to provide a first bending stiffness that allows the initial range of spinal bending and a second bending stiffness that restricts spinal bending beyond the initial range of spinal motion. One way to achieve both the first bending stiffness and the second bending stiffness is to configure the opening of the rod to have a lower bending moment of inertia I (sometimes referred to as the second moment of inertia or the area moment of inertia) through the initial range of spinal motion and a higher bending moment of inertia beyond the initial range of spinal motion.

Thus, with rod 104, dynamic portion 104A can allow a limited range of spinal bending, including flexion/extension motion. While the range of bending may vary from patient to patient, dynamic portion 104A can allow sufficient spinal bending to assist the adequate supply of nutrients to the disc in the supported portion of the spine. Movement beyond the initial range of motion is restricted by the dynamic portion 104A so as not to defeat the main purpose of the spine stabilization system. In some embodiments, dynamic portion 104A may refer to the portion of rod 104 that extends between bone fastener assembly 102 and universal clamp 105 as labeled in FIGURE 1. In some embodiments, dynamic portion 104A may extend beyond universal clamp 105 as depicted in FIGURE 1. In some embodiments, dynamic portion 104A may include a section for attaching universal clamp 105. The section may be smooth or textured and may have the same or different dimensions as other sections of dynamic portion 104A. In some embodiments, the section may have a larger diameter for improved contact with ligature 14. In some embodiments, the section may be smaller to prevent movement of ligature 14 along rod 104. In some embodiments, the section may have grooves for improved contact with ligature 14.

In some embodiments, rod 104 may have a cylindrical body having cannulated interior 211 and opening 220 extending spirally around a longitudinal axis of the cylindrical body. In one embodiment, opening 220 can form an interlocking pattern. In one embodiment, the interlocking pattern has a shape of or resembles a dog bone or puzzle. In one embodiment, opening 220 has a non-linear path. In one embodiment, dynamic portion 104A may comprise a biomaterial filling interior 211 in whole or in part. In one embodiment, dynamic portion 104A may be coated in whole or in part with a biomaterial. In one embodiment, a biomaterial at least partially fills opening 220. In one embodiment, a biomaterial may be a polymer. In one embodiment, a biomaterial is polycarbonate urethane. Other biomaterials are also possible.

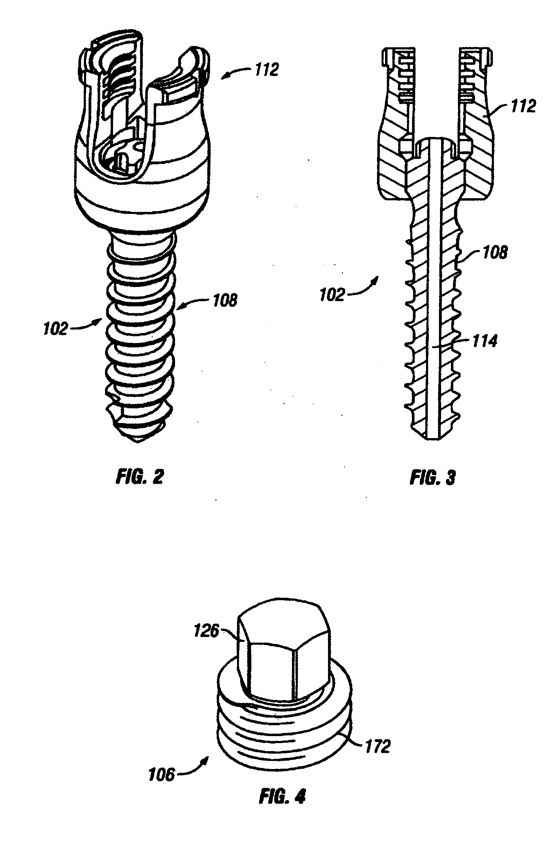

FIGURE 2 depicts a perspective view of bone fastener assembly 102 which may be used as an anchor to couple rod 104 to a vertebra. Those skilled in the art will appreciate that other anchors may be possible. Bone fastener assembly 102 may be constructed as a single component or may be constructed using various components.

FIGURE 3 depicts a cross-sectional representation of bone fastener 108 and collar 112 of bone fastener assembly 102. Bone fastener 108 of bone fastener assembly 102 may include passage 114. Bone fastener 108 may be cannulated (i.e., passage 114 may run through the full length of the bone fastener). A guide wire may be placed through passage 114 so that bone fastener 108 may be inserted into a vertebra at a desired location and in a desired angular orientation relative to the vertebra with limited or no visibility of the vertebra.

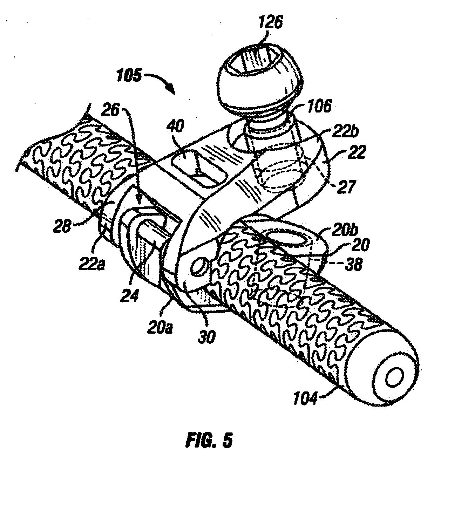

FIGURE 4 depicts one embodiment of closure member 106. Closure member 106 may be coupled to collar 112 of bone fastener assembly 102 to fix rod 104 positioned in collar 112 to bone fastener assembly 102. In some embodiments, closure member 106 may be cannulated. In certain embodiments, a closure member may have a solid central core. A closure member with a solid central core may allow more contact area between the closure member and a driver used to couple the closure member to a collar. A closure member with a solid central core may provide a more secure connection to a rod than a cannulated closure member by providing contact against the rod at a central portion of the closure member as well as near an edge of the closure member. Closure member 106 may include tool portion 126 and thread 172. Tool portion 126 may couple to a tool that allows closure member 106 to be positioned in collar 112. Tool portion 126 may include various configurations for engaging a tool. Tool portion 126 may be external, such as shown in FIGURE 4, or may be internal, such as shown in FIGURE 5. Male modified thread 172 may have a shape that complements the shape of a thread in arms of collar 112.

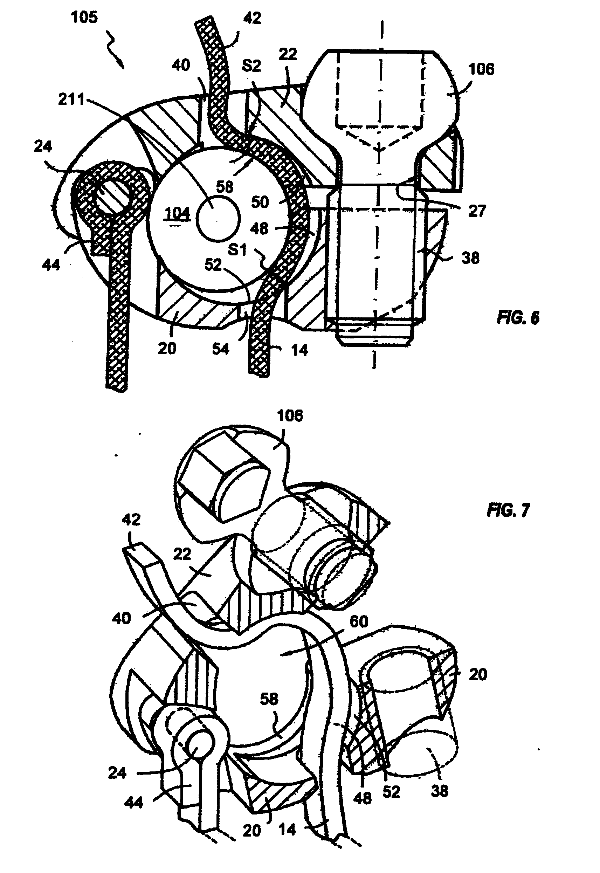

FIGURE 5 shows one embodiment of a portion of a spine stabilization system, specifically blocking body 105 mounted on a dynamic portion 104A of rod 104. Blocking body 105 comprises two longitudinal members, of which first longitudinal member 22 extends between a first end 22a and a second end 22b and second longitudinal member 20 extends between a first end 20a and a second end 20b. The two longitudinal members 22 and 20 may be pivoted together at their first ends 20a and 22a for the purposes of coupling blocking body 105 to rod 104. First end 22a of longitudinal member 22 has notch 26 with two opposite edges 28 and 30 and between which the first end 20a of second longitudinal member 20 may be inserted. Pivot pin 24 passes through the two first ends 20a and 22a and is free to rotate in at least one of ends 20a and/or 22a. The second end 22b of first longitudinal member 22 includes bore 27 into which closure member 106 may be inserted. The second end 20b of second longitudinal member 20 comprises a thread 38 which is aligned with bore 27 when the two longitudinal members are disposed facing each other, with the result that closure member 106 may be rotationally advanced into thread 38 in order to drive the second ends 20b and 22b of longitudinal members 20 and 22 towards each other. The consequences of advancing closure member 106 into thread 38, thereby coupling blocking body 105 to rod 104, are explained in more detail hereinafter. FIGURE 5 also shows a first orifice 40 (also referred to as exit passage 40) through which a ligature may be stretched. The method of connecting said ligature to blocking body 105 is described with reference to FIGURE 6.

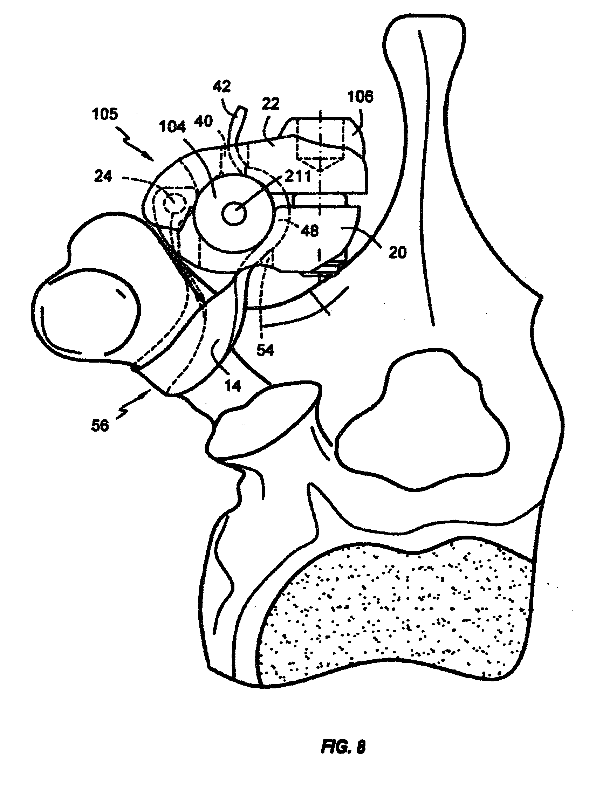

Referring to FIGURES 6-8, FIGURE 6 shows blocking body 105 consisting of first longitudinal member 22 and second longitudinal member 20, said longitudinal members 22 and 20 pivoting about pin 24 that joins them. The adjustable locking means may include closure member 106 passing through bore 27 and advanced into thread 38. to immobilize blocking body 105 relative to rod 104 and fix in position a portion of a ligature 14 shown in part in FIGURE 6. FIGURE 6 shows that second portion 58 of the middle part comes into contact with rod 104 and is adapted to bear on top of it and the first portion presses the free second end 42 of ligature 14 against rod 104. The adjustable locking means therefore drive longitudinal members 22 and 20 forcibly against rod 104 and simultaneously against ligature 14, which is also forcibly pressed against rod 104. In some embodiments, passage 48 has a section S1 in the vicinity of orifice 54 (also referred to as loop passage 54) greater than section S2 in the vicinity of first orifice 40, the section of passage 48 decreasing progressively in the direction from loop passage 54 to exit passage 40. Ligature 14 is therefore progressively compressed around a portion of rod 104 with a pressure that increases in the direction from loop passage 54 towards exit passage 40. Ligature 14 consists of an elongate flexible member capable of conforming to the contour of the parts that it must connect. Ligature 14 may be made from a flexible material such as polyester that may be lightly crushed locally to increase the friction forces, which may restrict movement of ligature 14 or immobilize it with a clamping effect. Ligature 14 has first end 44 that is ligated around pin 24 and free second end 42 that is inserted into passage 48 between rod 104 and internal walls 50 and 52 of longitudinal members 22 and 20 and external wall of rod 104. As shown in FIGURE 6, longitudinal member 20 may include second orifice 54 through which ligature 14 passes. Moreover, as shown in FIGURE 8, ligature 14 may be formed into loop 56 in which the transverse process is trapped. In some embodiments, ligature 14 may also loop around a lamina or some other non-pedicle portion of the spine such that blocking body 105 and rod 104 may be coupled to the spine.

As shown in FIGURE 7, which shows longitudinal member 22 at some angle relative to longitudinal member 20, the middle part has a first portion through which ligature 14 passes and second portion 58 adapted to bear directly on rod 104. In some embodiments, passage 48, which is symmetrical inside longitudinal member 20, is produced by a groove formed in each of the two facing faces of the middle parts of longitudinal members 22 and 20.

In some embodiments, the first portion of the middle part forms an edge with cylindrical symmetry and that the corresponding second portion of the middle part 58 of longitudinal member 20 forms a substantially cylindrical space 60 into which said rod 104 is inserted.

FIGURE 8 shows blocking body 105 mounted on a vertebra having a transverse process. Longitudinal members 22 and 20 securely couple blocking body 105 with rod 104 and press a portion of ligature 14 against rod 104. In FIGURE 8, flexible ligature 14 consists of a flexible strip of substantially constant width and thickness whose first end is ligated to pin 24. Ligature 14 may be passed around the transverse process of the vertebra and inserted in blocking body 105 via second orifice 54. The section of the flexible strip 14 is substantially rectangular so that, pin 24 and rod 104 being substantially perpendicular to the transverse process, ligature 14 may be partly twisted in order to insert it into passage 48 and between pin 24 and the point at which it contacts the transverse process. Blocking body 105 is fixed in position against the posterior wall of the vertebra despite these partially twisted portions, ligature 14 being forcibly tensioned by stretching free second end 42 of ligature 14.

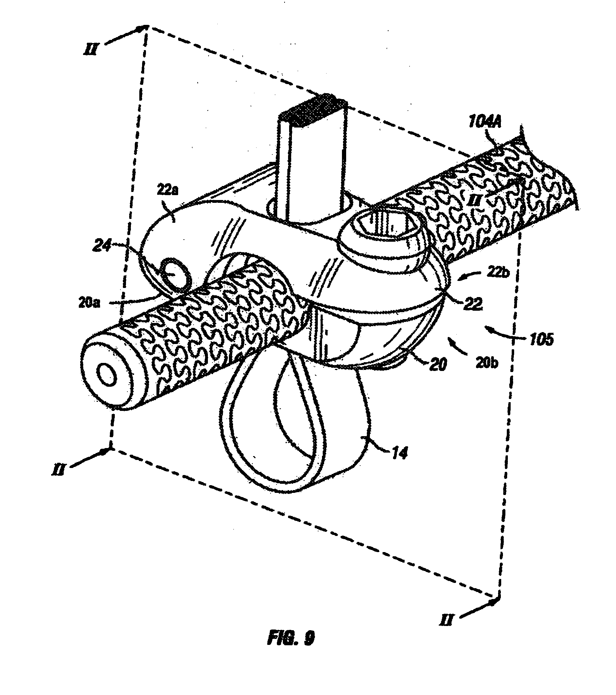

In some embodiments, flexible ligature 14 may not be ligated around pin 24 or otherwise fixed to blocking body 105. As shown in FIGURE 9, in one embodiment, a portion of a spine stabilization system may include blocking body 105 and flexible ligature 14 having two free ends. Flexible ligature 14 is of elongate shape and is capable of matching the outline of the parts it is to connect together. In this figure, there can also be seen dynamic portion 104A of rod 104 that is to be secured to the vertebra by means of blocking body 105. In the first embodiment, blocking body 105 includes longitudinal elements 22 and 20, each having a first end 22a, 20a and a second end 22b, 20b.

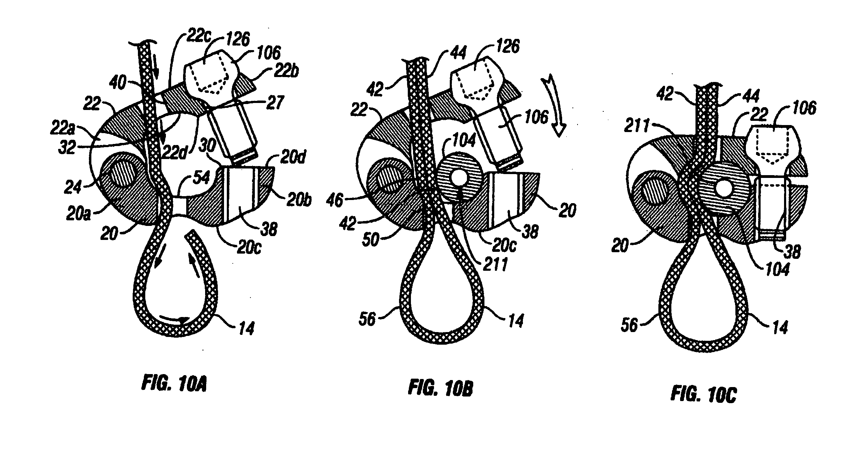

In FIGURE 10A, longitudinal elements 22 and 20 are hinged at their first ends 22a, 20a about pivot pin 24. In some embodiments, closure member 106 having head 106a that is engaged in bore 27 formed in the second end 22b of the longitudinal element 22 securely couples longitudinal elements 22 and 20. The second end 20b of the longitudinal element 20 is pierced by a tapped bore 38 for co-operating with the threaded shank 106b of closure member 106. Each longitudinal element 20, 22 has an outside face 20c, 22c and an inside face 20d, 22d. The longitudinal elements 20 and 22 are mounted in such a manner that the inside faces 20d, 22d of the longitudinal elements face each other. The inside faces 20d, 22d of the longitudinal elements 20 and 22 have respective mutually-facing recesses 30 and 32, each of substantially semi-cylindrical shape. The recesses 30 and 32 define walls which may be ruled surfaces having friction generator lines parallel to the pivot axis 24. Finally, slots 54 and 40 cause the bottoms of the recesses 30 and 32 to communicate with the outside faces 20c and 22c of the longitudinal elements 20 and 22. As explained below, the recesses 30 and 32 are for receiving rod 104 together with a strand of ligature 14, the slots 54 and 40 serving to pass ligature 14.

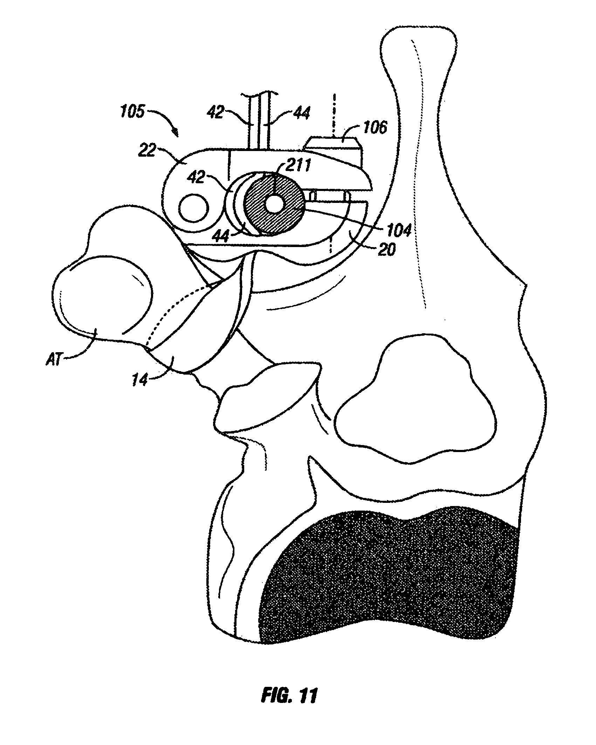

With reference to FIGURES 10A, 10B, 10C and 11 there follows an explanation of how blocking body 105 and conformable ligature 14 may be used to couple rod 104 to a portion of the spine, such as a lamina or other non-pedicle portion.

As depicted in FIGURE 10A, in some embodiments, longitudinal elements 20 and 22 may be positioned in an open configuration (or spaced-apart position), in which the threaded shank of closure member 106 is disengaged from bore 38. Ligature 14 may be passed or ligated around a non-pedicle portion of a vertebra and passed through loop passage 54 and out exit passage 40 of longitudinal elements 20 and 22 such that the vertebra is coupled to blocking body 105. Rod 104 may then be introduced into recesses 30 and 32 of longitudinal elements 20 and 22 so that strands 42 and 44 of ligature 14 are disposed between the inside wall of recesses 30 and 32 and the side face of rod 104. These two surfaces define passageway 48 for passing ligature 14 and having portions 42 and 44 of ligature 14 placed therein.

As shown in FIGURE 10B, portions of ligature 14 define a portion of ligature 14 that forms loop 56 that extends beyond the outside face 20c of the longitudinal element 20, and also two free portions 42 and 44 that extend beyond the outside face 22c of the longitudinal element 22. When the longitudinal elements 20 and 22 are in an open configuration (i.e., spaced apart as shown in FIGURE 10A or 10B), ligature 14 can slide freely along passageway 48 through recesses 30 and 32. Once ligature 14 is placed around the transverse process, a rib, a lamina, a portion of the posterior arc of a vertebra or the like, threaded shank of closure member 106 may be rotationally advanced into tapped bore 38, causing longitudinal element 22 to come progressively closer to longitudinal element 20. This approach simultaneously reduces the section of passageway 48 in which portions 42 and 44 of ligature 14 are engaged, and simultaneously introduces a coefficient of friction between ligature 14, rod 104 and walls of the recesses 30 and 32. Nevertheless, it is still possible for traction to be applied on the free ends 42 and 44 of ligature 14 until sufficient tension is obtained in ligature 14 around the non-pedicle portion of the vertebra. Once the tension in ligature 14 reaches a desired level, closure member 106 may be further advanced in tapped bore 38 to lock longitudinal elements 20 and 22 together. Advantageously, the portions 42 and 44 of ligature 14 are pinched between rod 104 and the wall of the recesses 30 and 32. In this locking position, rod 104 is thus secured to ligature 14 via blocking body 105. Advantageously, because the surgeon exerts traction only on the free ends 42 and 44 of ligature 14, there is no risk of ligature 14 forming a mass under the bottom face of the transverse process, lamina, rib, etc., thus guaranteeing that effective fastening is provided with the lamina, transverse process, rib or the like. FIGURE 11 depicts a face view where reference AT identifies the transverse process. As shown in FIGURE 11, conformable ligature 14 may be passed around a portion of the spine and captured in blocking body 105, and blocking body 105 may also capture rod 104 such that rod 104 is coupled to a portion of the spine.

In the above description, both of the portions 42 and 44 of ligature 14 are disposed in the recesses 30 and 32 on the same side of rod 104. In some embodiments, the portions 42 and 44 of ligature 14 may be placed on opposite sides of rod 104. Under such circumstances, it should be considered that the outside face of rod 104 and the inside walls of the recesses 30 and 32 define two passageways, respectively for passing each of the portions 42 and 44 of ligature 14.

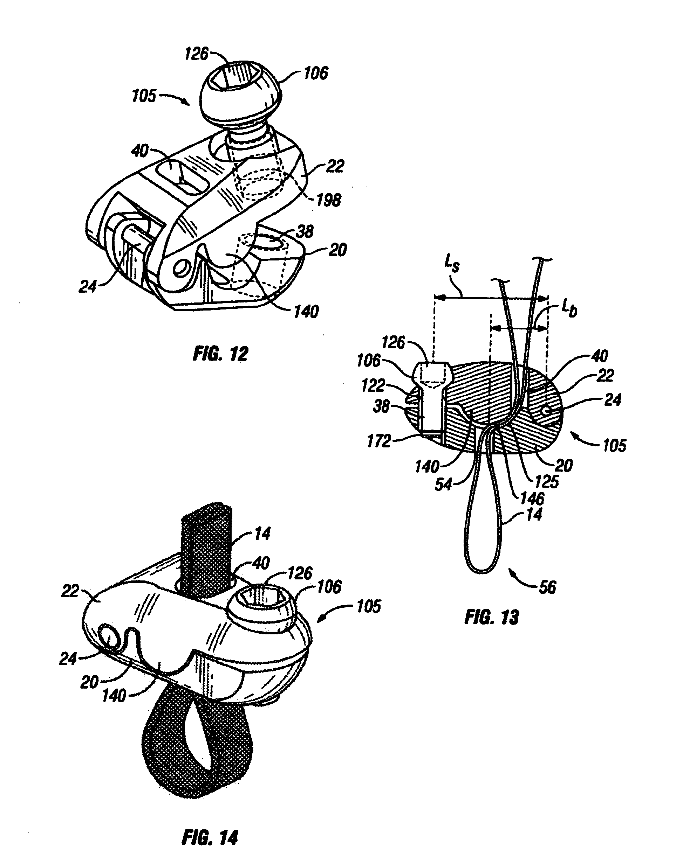

FIGURES 12-14 illustrate an example of blocking body 105 that provides a hinged blocking body 105 offset from ligature 14 that also does not require a rod to capture ligature 14, and which can provide certain advantages over other embodiments. FIGURE 12 shows blocking body 105 with longitudinal element 20 hingedly connected via hinge pin 24 to longitudinal element 22. Conformable ligature 14 can pass through loop passage 54 in longitudinal element 20 of blocking body 105 to form loop 56 extending from longitudinal element 20 of blocking body 105, further passed through a passage formed between first surface 146 of compression member 140 and inner surface 125 of blocking body 105, and then passed through exit passage 40 in longitudinal element 22. As with every embodiment or example described, ligature 14 may get to this desired configuration (with a loop portion extending from blocking body 105) in a variety of ways. An advantage to this example is the low profile possible due to the configuration of closure member 106 offset from compression member 140.

In various examples, hinge pin 24 connects longitudinal element 20 to longitudinal element 22 in either a permanent manner or alternatively the hinged connection may be disconnectable. The hinged connection can be formed so as to allow two-way hinged motion for engaging or disengaging longitudinal element 22 from longitudinal element 20. In an alternative example, the hinged connection may allow one-way hinged motion for engaging longitudinal element 22 from longitudinal element 20 but may subsequently prevent longitudinal element 22 from disengaging longitudinal element 20. In various examples longitudinal element 22 and/or longitudinal element 20 may allow hinged motion between a selected arclength. For example, longitudinal element 22 and longitudinal element 20 may move through an arc of approximately 180 degrees.

As further shown in FIGURES 12-14, closure member 106 may be used to close blocking body 105 in a manner to hold ligature 14 in a relatively or completely stable position relative to blocking body 105. Closure member 106 of FIGURE 13 is shown having external threads 172 for engaging bore 38 in longitudinal member 20 of blocking body 105. In one example, closure member 106 may be positioned within longitudinal element 22 such that closure member 106 is free to rotate in longitudinal element 22 to join longitudinal element 22 with longitudinal element 20, but may not be removed from longitudinal element 22 after such engagement. In other words, in some examples, closure member 106 may be rotated to engage threads on longitudinal element 20 to collapse longitudinal element 22 and longitudinal element 20, and the direction of rotation may be reversed to disengage closure member 106 from longitudinal element 20 but closure member 106 may not be removed from longitudinal element 22. In one example, closure member 106 can be rotatably advanced in longitudinal element 20 such that closure member 106 is free to rotate in longitudinal element 22 but may not be removed from longitudinal element 22, which can provide the advantage of reducing the risk of having loose hardware (which can be lost inside a patient during surgery) associated with blocking body 105.

FIGURE 13 depicts a cross-sectional side view of a portion of the example of blocking body 105 depicted in FIGURE 12, in which conformable ligature 14 may be passed through loop passage 54 in longitudinal element 20 to form loop 56 extending from longitudinal element 20 of blocking body 105, passed through a passage formed by first surface 146 of compression member 140 and inner surface 125 of blocking body 105, and extend from orifice 40 in longitudinal element 22. As shown in FIGURE 13, longitudinal element 20 and longitudinal element 22 rotate about hinge pin 24 to open or close blocking body 105. Tool portion 126 on closure member 106 may be rotated so threads 172 on closure member 106 engage threads 122 in longitudinal element 22. Once closure member 106 reaches a selected point, longitudinal element 22 and longitudinal element 20 compress ligature 14 to impinge movement of ligature 14 relative to blocking body 105.

First surface 146 of compression member 140 and inner surface 125 of blocking body 105 provide a passage way through blocking body 105. In some examples, inner surface 125 may be located on longitudinal element 20 and first surface 146 of compression member 140 may be located on longitudinal element 22 as depicted in FIGURE 13. In some examples, inner surface 125 may be located on longitudinal element 22 and first surface 146 of compression member 140 may be located on longitudinal element 20.

Closure member 106 may be offset from compression member 140 such that threaded engagement of threads 172 of closure member 106 with engagement bore 38 of blocking body 105 may indirectly apply compression to compression member 140. In other words, compression member 140 may be positioned some distance L_{b} from hinge pin 24 and closure member 106 may be positioned some distance Lₛ from hinge pin 24. Compression of compression member 140 onto conformable ligature 14 may be accomplished by rotationally engaging threads 172 with threads in bore 38 to advance closure member 106 in longitudinal element 20 such that longitudinal element 22 may be leveraged around the fulcrum created by hinge pin 24. An advantage to one example uses the mechanical advantage of Lₛ / L_{b} to apply compression forces on conformable ligature 14. Another advantage to one example is the ability for the surgeon to apply large compression forces to conformable ligature 14 due to the mechanical advantage based on the position of hinge pin 24, compression member 140, and closure member 106. The compression forces available may also be based on the radius of curvature of compression member 140, the size or pitch of threads 172, and/or the size of hinge pin 24. Another advantage may be the precision in which a friction coefficient may be selected between conformable ligature 14 and blocking body 105. In some examples, the pitch, shank diameter, or other dimensions of closure member 106 may enable control of the application of compression. For example, a large number of threads per inch may allow more compression due to the mechanical advantage of threads 172 engaging with threads in bore 38, and the application may be more controlled due to the greater angular rotation needed to advance closure member 106 the same distance as closure members 106 having lower numbers of threads per inch. Another advantage to this examples relates to the outer surface of longitudinal element 22 and/or longitudinal element 22. Because blocking body 105 can achieve a mechanical advantage through the use of hinge pin 24, closure member 106 may be made smaller than prior art approaches, which allows blocking body 105 to have a smaller opening bore 38. As shown in FIGURE 13, longitudinal element 22 may have an outer surface that is curved, which may reduce pain, discomfort, or other undesirable effects that result from using an angular implant

FIGURE 14 depicts a perspective view of the example of FIGURES 12 and 13 shown in a closed configuration, where ligature 14 is held completely or substantially in place relative to blocking body 105. One advantage to this type of example may be the ability to pass conformable ligature 14 around one or more bones, tendons, muscles, rods, plates, screws, or other structures in the body, pass conformable ligature 14 through blocking body 105 out a single exit passage 40, and engage closure member 106 on blocking body 105, but offset from conformable ligature 14 and/or compression member 140. One advantage may be that a tensioning tool may not need an opening in its distal end because closure member 106 (e.g., tool portions 126) may be accessed from outside the tensioning tool.

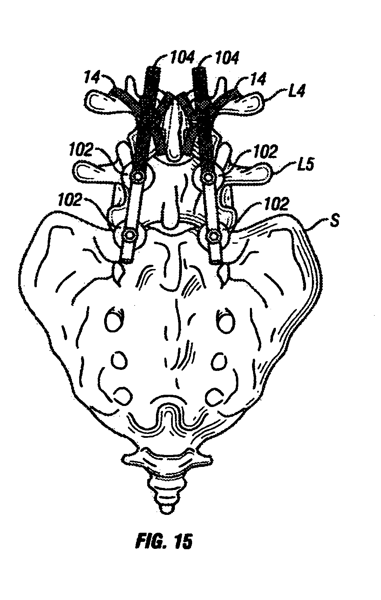

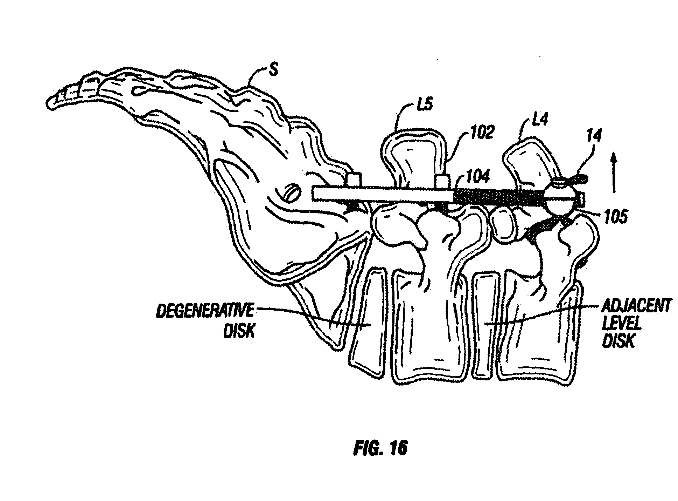

FIGURES 15 and 16 depict posterior and sagittal views of a portion of the spine in which bone fastener assemblies 102, blocking body 105, and rod 104 may be useful for preventing adjacent level disc disease. In some embodiments, bone fastener assemblies 102 may be implanted in lumbar vertebra L5 and sacrum S to stabilize the spine around a degenerative disc located between L5 and S. In some embodiments, bone fastener assemblies 102 may be inserted through an incision in the skin and implanted using Minimally invasive Surgery (MIS) techniques. Ligature 14 may be passed around a portion of a vertebra. Rod 104 having a rigid portion and a dynamic portion may be connected to bone fastener assemblies 102, and ligature 14 may be passed around rod 104 and a non-pedicle portion of a vertebra and secured by blocking body 105. As depicted in FIGURE 16, ligature 14 may be connected to a portion of lumbar vertebra L4 so that dynamic portion 104A of rod 104 spans the vertebral disc between L4 and L5 (i.e. the adjacent level disc). An advantage to this embodiment may be that the Sacrum (S) and Lumbar Vertebra V (L5) may be rigidly connected, but L5 and Lumbar Vertebra IV (L4) may be dynamically connected such that L4 can move relative to L5, which may prevent or slow the effects of Adjacent Level Disease in the disc located between L4 and L5.

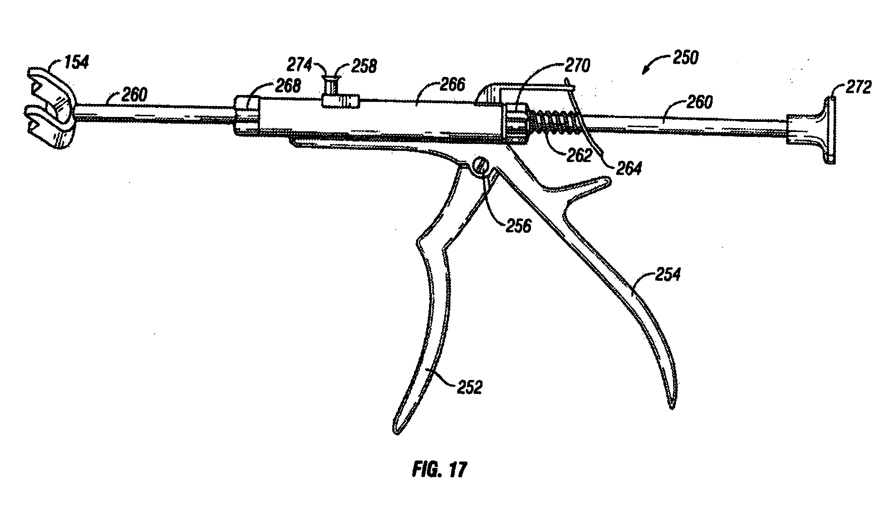

FIGURE 17 depicts a side view of a portion of one example of tensioning tool 250, which may be used to apply tension to conformable ligature 14 to engage rod 104, a portion of a vertebra, or some other device or bone. As shown in FIGURE 17, tensioning tool 250 includes tool body 266 for engaging conformable ligature 14, longitudinal member 260 for advancement in tool body 266, and distal end 154 for engagement with blocking body 105. As shown in FIGURE 17, tool body 266 includes attachment point 274 (with flange 258) for connection to ligature 14, fixed handle 254, movable handle 252 for rotation about axis 256, return spring 262, catch mechanism 264, return spring adjustment member 270, and spring adjustment member 268.

Attachment point 274 can attach first and second ends of conformable ligature 14 to tensioning tool 250. In some embodiments, attachment point 274 may include flange 258 for preventing first and second ends of conformable ligature 14 from detaching from tensioning tool 250. Distal end 154 of tensioning tool 250 may engage to a portion of blocking body 105. Fixed handle 254 may be gripped by a surgeon, movable handle 252 may be rotated about axis 256, such as by squeezing movable handle 252, to advance longitudinal member 260 through tool body 266 a selected distance. Advancing longitudinal member 260 to move blocking body 105 away from tool body 266 while maintaining first and second ends of conformable ligature 14 on attachment point 274 applies tension to conformable ligature 14. In some examples, the selected distance longitudinal member 260 advances through tool body 266 may be proportional to the tension applied to conformable ligature 14.

Tool body 266 may include return spring 262, catch mechanism 264, and return spring adjustment member 270 for controlling the distance that longitudinal member 260 is allowed to return when movable handle 252 is released. In some embodiments, return spring 262 may bias catch mechanism 264 such that movement is permitted in one direction only. Return spring 262 may bias catch mechanism 264 such that longitudinal member 260 may only move forward through tool body 266. Advantageously, return spring 262 may ensure that a surgeon does not inadvertently relieve tension from conformable ligature 14. In other words, tensioning tool 250 may have a default configuration for tensioning conformable ligature 14. Actuating catch mechanism 264 (such as a surgeon pressing on catch mechanism 264 with a thumb) may change the positioning of catch mechanism 264 such that movement of longitudinal member 260 is permitted in a reverse direction as well. Movement of longitudinal member 260 in a reverse direction may include changing the positioning of catch mechanism 264 in relation to longitudinal member 260 as well as pulling in a reverse direction on grasping member 272.

Tensioning tool 250 may include spring adjustment member 268 for adjusting the compression on a spring (not shown) in body 266. Rotating spring adjustment member 268 one direction, spring adjustment member 268 may be advanced some distance into body 266 such that a spring may be compressed. Rotating spring adjustment member 268 in the other direction, spring adjustment member 268 may be advanced some distance out of body 266 such that compression forces on the spring may be relieved. By changing the compression forces on the spring, the spring may exert more or less force on longitudinal member 260, which may affect how much tension can be applied to the ends of conformable ligature 14.

Ligature 14 may be passed around rods 104, bone fastener assemblies 102, vertebrae (such as L4), and other tendons, muscles, plates or other anatomical or implanted structures and the ends of ligature 14 may be passed via orifice 54 into a portion of blocking body 105, such that a loop is formed extending from a first portion of blocking body 105. income First and second ends of ligature 14 may be passed through a passage in blocking body 105. A passage may be formed by inner surface 125 of blocking body 105 and first surface 146 of compression member 140 or rod 104. In some embodiments, first and second ends of ligature 14 may exit by passing out of one or more orifices 40 in blocking body 105.

Distal end 154 of tensioning tool 250 engages blocking body 105. In some embodiments, distal end 154 of longitudinal member 260 may conform to the shape or profile of blocking body 105. Distal end 154 of longitudinal member 260 may be configured with features for engaging one or more features on blocking body 105. First and/or second ends of ligature 14 may be attached to tensioning tool 250. In some embodiments, first and/or second ends of ligature 14 may be attached to attachment point 274 located on tool body 266. In some embodiments, movable handle 252 of tensioning tool 250 may be rotated about axis 256 to advance longitudinal member 260 through tool body 266. The advancement of longitudinal member 260 through tensioning tool 250 moves attachment point 274 away from blocking body 105, pulling ends of ligature 14 to decrease the size of the loop, and further advancement tensions ligature 14. The tension applied to ligature 14 may be sufficient to hold one or more structures in a desired position. The tension applied to ligature 14 may be sufficient to hold rod 104 relative to a vertebra to provide dynamic stabilization of the vertebra in relation to adjacent vertebrae.

Once an appropriate tension has been applied to ligature 14, closure member 106 may be actuated to create a friction force to restrict movement of ligature 14 relative to blocking body 105 or to impinge ligature 14 in blocking body 105. Closure member 106 may be pre-installed in blocking body 105. Closure member 106 may be inserted in blocking body 105 after engagement of blocking body 105 by tensioning tool 250. Closure member 106 may be inserted through distal end 154 of longitudinal member 260 into blocking body 105.

Once closure member 106 has engaged threads in bore 38 in blocking body 105 to provide a desired friction force to impinge ligature 14 in blocking body 105, first and second ends of ligature 14 may be disconnected from tensioning tool 250. Once ligature 14 has been disconnected from tensioning tool 250, tensioning tool 250 may be disengaged from blocking body 105.

Passing may include going into, through, or out of a structure. Passing may include going over, under, or around a structure. Passing may include crossing over other ligatures 14 or portions of ligatures 14. Ligature 14 may be passed around a non-pedicle portion of a vertebra before passing both ends through blocking body 105, or one end may be passed through blocking body 105 and the second end may be passed around a non pedicle portion of a vertebra and then passed through blocking body 105. Thus, tensioning tool 250 may be used in invasive or non-invasive surgical procedures to couple dynamic portion 104A of rod 104 to non-pedicle portions of the spine.

Various instruments may be used in a minimally invasive procedure to position bone fastener assemblies 102 in a patient for coupling rigid portion 104B of rod 104 to pedicles of a spine to form a part of a spinal stabilization system. The instruments may include, but are not limited to, positioning needles, guide wires, dilators, bone awls, bone taps, sleeves, drivers, tissue wedges, rod length estimating tools, tensioning tools, mallets, tissue retractors, and tissue dilators. The instruments may be provided in an instrumentation set. The instrumentation set may also include components of the spinal stabilization system. The components of the spinal stabilization system may include, but are not limited to, bone fastener assemblies of various sizes and/or lengths, rods, clamps, conformable ligatures and closure members.

Instruments used to install a spinal stabilization system may be made of materials including, but not limited to, stainless steel, titanium, titanium alloys, ceramics, and/or polymers. Some instruments may be autoclaved and/or chemically sterilized. Some instruments may include components that cannot be autoclaved or chemically sterilized. Components of instruments that cannot be autoclaved or chemically sterilized may be made of sterile materials. The sterile materials may be placed in working relation to other parts of the instrument that have been sterilized.

A targeting needle may be used to locate an entry point in a vertebral body for a bone fastener of a bone fastener assembly. Scale markings may be used to approximate a length of a bone fastener needed for a vertebra. A guide wire may pass down a shaft of a targeting needle outer housing. A guide wire may be from about 15 cm to about 65 cm in length. Guide wires provided in an instrumentation set are about 46 cm in length. The length of the guide wire may allow a surgeon and/or assistants to hold at least one portion of the guide wire at all times when the guide wire is inserted into vertebral bone, even during insertion, use, and removal of instruments along a length of the guide wire. A guide wire that can be held continuously during a surgical procedure may inhibit removal or advancement of the guide wire from a desired position during a minimally invasive surgical procedure.

Dilators may be used during a minimally invasive surgical procedure to push aside tissue and create space to access vertebral bone. Four tissue dilators of increasing diameter may be used to establish sufficient working space to accommodate instruments and spinal stabilization system components. Especially for a mid-vertebra, only three dilators may be needed to form sufficient working space. Dilators in an instrumentation set may increase in diameter incrementally by a selected amount. For example, outside diameters of dilators in an instrumentation set may increase sequentially by increments of about 0.5 mm. A bone awl may be used to breach cortical bone of a pedicle. A guide wire that is inserted in vertebral bone in a desired orientation may be inserted through the bone awl. The bone awl may be moved down the guide wire so that the tip of the bone awl contacts the pedicle. During some surgical procedures, an impact force may be needed for the bone awl to breach cortical bone. A guide wire may be removed, the bone awl may be used to breach cortical bone, and the guide wire may be reinserted. A small dilator may be placed over the portion of the guide wire extending from the bone awl so that a first end of the dilator contacts the bone awl. A mallet or other impact device may be used against a second end of the dilator so that the bone awl breaches cortical bone of the vertebra. The dilator may be removed from the bone awl and contact with the guide wire may be reestablished.

A bone tap may be used to form a threaded passage of a desired depth through a pedicle and into a vertebral body. X-ray monitoring of a depth of a tap portion of known length may allow a medical practitioner to assess a depth of a hole tapped in a bone. The hole may be tapped to accommodate a bone fastener of a desired length. A bone fastener may be chosen to accommodate a hole tapped to a desired depth.

A guide wire positioned in vertebral bone may be held near a top of a dilator inserted over the guide wire at a surgical site.

A detachable member may be used as a guide to install bone fasteners of a bone fastener assembly in vertebral bone. A detachable member may be coupled to a collar of a bone fastener assembly. A distal end of a detachable member may be tapered or angled to reduce bulk at a surgical site. Instruments may be inserted into the detachable member to manipulate the bone fastener assembly. Movement of the detachable member may alter an orientation of a collar relative to a bone fastener of the bone fastener assembly. In some embodiments, a detachable member may be used as a retractor during a spinal stabilization procedure.

A detachable member for a vertebral stabilization system may include one or more channels in a wall of the detachable member to allow access to an adjacent vertebra. For some vertebral stabilization procedures, only single-channel detachable members having a single channel in a wall of the detachable member may be used. For other vertebral stabilization procedures, one or more multi-channel detachable members having two or more channels in a wall of the detachable member may be used. Channels may provide flexibility to or enhance flexibility of a multi-channel detachable member. A proximal portion of a multi-channel detachable member may have a solid circumference. A region of solid circumference in a multi-channel detachable member may enhance stability of the multi-channel detachable member. A multi-channel detachable member may be longer than a single-channel detachable member.

A detachable member used at a middle vertebra in a multi-level stabilization procedure may be a multi-channel detachable member. Channels in a multi-channel detachable member may allow access to adjacent vertebrae from a middle vertebra. A detachable member used at an end vertebra of a multi-level stabilization system may be a single-channel detachable member or a multi-channel detachable member. A system for coupling a bone fastener assembly to a multi-channel detachable member may include a limiter that inhibits spreading of arms of the detachable member to inhibit release of the bone fastener assembly from the detachable member.

A channel in a wall of a detachable member may allow access to a vertebra that is to be stabilized with a spinal stabilization system being formed. A single-channel detachable member may be coupled to a bone fastener assembly to be inserted into vertebral bone of a first vertebra. The single-channel detachable member may allow access to a second vertebra from the first vertebra. A multi-channel detachable member may be coupled to a bone fastener assembly to be inserted into vertebral bone of a first vertebra. The multi-channel detachable member may allow access from the first vertebra to adjacent vertebrae.

Instruments may access a bone fastener assembly through a passage in a detachable member. A channel in a wall of a detachable member may extend a full length of the detachable member. Especially in embodiments of multi-channel detachable members, a channel in a wall of a detachable member may extend only a portion of the length of the detachable member. A channel in a wall of a detachable member may extend 25%, 50%, 75%, 80%, 90%, 95% or more of the length of the detachable member. A channel may extend to a distal end of a detachable member such that a rod inserted in the channel may pass from the detachable member into a slot of a collar of a bone fastener assembly coupled to the detachable member.

A channel in a detachable member may be any of a variety of shapes. A channel may have a width that exceeds a width or diameter of a rod that is to be inserted in the channel. In some embodiments, a channel may be a linear opening parallel to a longitudinal axis of the detachable member. A channel may have a non-linear shape including, but not limited to, a helical pattern, an arc, an "L" shape, or an "S" shape. A non-linear channel may allow a rod to travel along a predetermined path. Adjacent detachable members may include channels with matching profiles, allowing ends of a rod to follow similar paths down the detachable member channels.

Movable members may extend through portions of a detachable member proximate a channel in the detachable member. Movable members may engage notches in a collar to establish a radial orientation of the detachable member on the collar and/or to inhibit rotation of the collar relative to the detachable member. A distal end of a movable member may be flat, curved, or angled. In some embodiments, a distal end of a movable member may be threaded. In other embodiments, a distal end of a movable member may be a projection that engages an opening in a collar. An upper surface of a collar and/or a surface of a distal end of a movable member may be textured to inhibit rotation of the collar relative to the detachable member. A proximal end of a movable member may include a tool engaging portion. A tool engaging portion may include, but is not limited to, a hex section, a hexalobular section, a tapered section, a bead, a knot, a keyed opening, a coating, a threading, and/or a roughened surface for engaging a drive that rotates or otherwise displaces the movable member.

A cross section transverse to a longitudinal axis of a detachable member may have shapes including, but not limited to, circular, ovoid, square, pentagonal, hexagonal, and combinations thereof. In some embodiments, a detachable member may be hollow. In certain embodiments, a thickness of a hollow detachable member may be uniform. A thickness of a hollow detachable member may vary along the length of the detachable member. A detachable member with a passage extending longitudinally from a first end of the detachable member to a second end of the detachable member may be referred to as a "sleeve".

A detachable member may be coupled to a collar of a bone fastener assembly in various ways. When a detachable member is coupled to a collar, rotation and translation of the detachable member relative to the collar may be inhibited. A system used to couple a detachable member and collar should be simple, inexpensive to implement, and should not significantly weaken the mechanical strength of the collar and/or the detachable member. Detachable members may be coupled to collars using various coupling systems including, but not limited to, flanges, threaded connections, interlocking connections (e.g., ratcheting connection systems), and/or interference fits.

In an example of an interlocking connection system, a detachable member may include an opposing pair of deflectable arms. Each deflectable arm may include a tooth. The deflectable arms may be forced outwards during coupling of a collar to the detachable member. When the collar is coupled to the detachable member, the deflectable arms may be positioned in channels in the collar, with the teeth positioned in indentions in the collar. The presence of the deflectable arms in the channels of the collar may inhibit rotation and translation of the detachable member relative to the collar. Separation of the detachable member from the collar may be achieved by insertion of an expander in the detachable member. The expander may be used to force the deflectable arms outwards and expel the teeth from the indentions.

Detachable members may be of various lengths. Detachable members of different lengths may be used in the same surgical procedure. A detachable member length used in a spinal stabilization procedure may be determined by a patient's anatomy. Detachable members may be just short enough to allow manipulation by a medical practitioner above an incision in a patient. Detachable members may be about 3.5 to about 11.5 cm long. For example, a single-channel detachable member may be about 10 cm long. In some embodiments, detachable members may be about 11.5 cm to about 14 cm long. For example, a single-channel or a multi-channel detachable member may be about 12.5 cm long. A multi-channel detachable member may be longer than a single-channel detachable member. A multi-channel detachable member may be at least about 15 cm long. For example, a multi-channel detachable member may be about 16 cm long. Detachable members that are too long may require a longer incision and/or a larger tissue plane for insertion of a spinal stabilization system. Insertion of a rod may be more difficult with detachable members that are longer than necessary. Detachable members with excess length may be bulky and hard to manipulate during a surgical procedure.

A detachable member may be flexible over its entire length or include a flexible portion near a proximal end of the detachable member. A flexible portion may allow positioning of a proximal portion of a detachable member in a desired location. A flexible portion may be produced from any of various materials including, but not limited to, a surgical grade plastic, rubber, or metal. A flexible portion may be formed of various elements, including, but not limited to, a tube, a channel, or a plurality of linked segments.

When bone fasteners of polyaxial bone fastener assemblies are positioned in vertebral bone, detachable members coupled to collars of the bone fastener assemblies may be moved in desired positions. During surgery, a detachable member in a patient may be oriented towards an adjacent vertebra that is to be stabilized to reduce the required incision size. Channels of the detachable members may be aligned so that a rod may be positioned in collars of the bone fastener assemblies. Channels of the detachable members may be aligned so that a rod may be moved down the detachable members and into collars of the bone fastener assemblies.

After a bone fastener assembly is coupled to a detachable member, a driver may be coupled to a bone fastener of the bone fastener assembly. The driver may be used to insert the bone fastener into vertebral bone.

During a minimally invasive surgical procedure, a plane may be created in tissue from a first vertebra to a second vertebra. A rod may be positioned in the plane during the surgical procedure. In some embodiments, a tissue plane may be formed using a targeting needle. The targeting needle may be positioned at the first vertebra. The distal end of the needle may be moved toward the second vertebra to form the plane while maintaining a position of the needle at a surface of the skin. The needle may be moved back and forth a number of times to clearly establish the plane. Care may need to be taken to avoid bending the targeting needle during establishment of the plane.

A tissue wedge may be used to form a plane in tissue between a first vertebra and a second vertebra.

An estimating tool may be used to estimate a distance between bone fastener assemblies anchored in vertebrae. The bone fastener assemblies may be part of a spinal stabilization system. The distance estimated by an estimating tool may be used to determine a desired length of a rod to be positioned in collars of the anchored bone fastener assemblies.

A rod positioner may be used to guide a rod through detachable members and to position the rod in collars proximate pedicles of vertebrae.

In some cases, pressure supplied to a rod with a rod positioner may not be sufficient to seat the rod in a collar. A seater may be used in conjunction with a rod positioner to maneuver a rod into one or more collars. During some procedures, a rod positioner may be removed from the rod before using the seater. During some procedures, the rod positioner may remain attached to the rod until closure members are secured to bone fastener assemblies to form a spinal stabilization system. A user may apply downward force with handle 350 to seat the rod in a collar as the rod positioner is used to guide the rod into position. After a rod has been positioned and seated in collars as desired, closure members may be used to secure the rod to the collars.

Minimally invasive procedures may involve locating a surgical site and a position for a single skin incision to access the surgical site. The incision may be located above and between (e.g., centrally between) vertebrae to be stabilized. An opening under the skin may be enlarged to exceed the size of the skin incision. Movement and/or stretching of the incision, bending of a rod, and angulation of collars of bone fastener assemblies may allow the length of the incision and/or the area of a tissue plane to be minimized. Minimally invasive insertion of a spinal stabilization system may not be visualized. Insertion of a spinal stabilization system may be a top-loading, mini-opening, muscle-splitting, screw fixation technique.

Insertion of a spinal stabilization system may include gradually increasing the diameter of an opening formed in a pedicle and/or vertebral body to accept a bone fastener assembly. For example, a targeting needle may have outer diameter of about D. A bone awl inserted after the targeting needle may have an outer diameter incrementally larger than the outer diameter of the targeting needle. As used herein, an incrementally larger diameter may be large enough to allow a snug but adjustable fit. For example, the bone awl may have outer diameter of about (D+x). A tap portion of a bone tap inserted after the bone awl may have a minor diameter of about (D+2x). A bone fastener may have a minor diameter of about (D+3x). In some embodiments, x may be between about 0.1 mm and about 1.0 mm. For example, x may be about 0.5 mm. Incremental sizing of the targeting needle, bone awl, tap, and bone fastener may promote a proper fit of the bone fastener in the vertebra to be stabilized.

In an example of a spinal stabilization system insertion method, the patient may be placed in a prone position on a radiolucent table with clearance available for a C-arm of a fluoroscope. For example, a Jackson table with a radiolucent Wilson frame attachment may be used. The ability to obtain high quality images is very important. Bolsters, frames, and pads may be inspected for radiolucency prior to the operation. Placing the patient in a knee-chest position (e.g., using an Andrews table) should be avoided. Care should be taken to avoid placing the patient's spine in kyphosis during positioning of the patient.

The C-arm of the fluoroscope should be able to freely rotate between the anteroposterior, lateral, and oblique positions for optimal visualization of pedicle anatomy during the procedure. The arm should be rotated through a full range of motion prior to beginning the procedure to ensure that there is no obstruction or radio-opaque object in the way. The fluoroscope may be positioned so that Ferguson views and "bullseye" views are obtainable. Once the patient is positioned and the ability to obtain fluoroscopic images of the target levels for instrumentation has been confirmed, the patient may be prepared and draped sterilely.

For most of the lumbar region, the vertebral pedicle is an obliquely oriented cylindrical corridor. The angulation varies by approximately 5 degrees per level (e.g., L1: 5 degrees; L5: 25 degrees). A pre-operative fine-cut computed tomography image may be examined to determine any unique anatomy of the patient. Acquiring the pedicle in the most lateral and superior quadrant of the pedicle may be desirable to avoid the overriding facet during a minimally invasive procedure. A lateral entry point may allow for better screw convergence as well as less interference with the superior adjacent level facet joint. A targeting needle may be passed in a medial and inferior trajectory, thus following the natural pathway of the pedicle. Frequent fluoroscopic inspection in both an anteroposterior and lateral plane may ensure proper passage of the needle as the needle is inserted into vertebral bone.

Various techniques may be used to plan the skin incisions and entry points. In one embodiment, the planning sequence for a single-level stabilization may include the following four steps. First, an anteroposterior image may be obtained with the spinous processes centered at the target vertebral bodies. Vertical lines passing through midpoints of pedicles that are to receive bone fasteners may be marked on the patient. The lines do not represent skin entry points. The lines are markers of pedicle entry points used to estimate angles at which targeting needles to be inserted to contact the pedicles. In some embodiments, sets of vertical lines may be drawn corresponding to the lateral edges of the pedicles instead of lines corresponding to the midpoints of the pedicles.

Second, horizontal lines may be marked approximately through the centers of the pedicles (mid-pedicle lines) on the patient. In some embodiments, the lines may be drawn on the superior side of the center axes (superior to the mid-pedicle

Third, an oblique or "bullseye" view (i.e., down a longitudinal axis of a pedicle) may be obtained on each side of the patient for each pedicle that is to be stabilized. Vertical oblique view lines may be marked on the skin at the midpoints of each of the pedicles that are to receive a bone fastener. The oblique view lines may be drawn in a different color than the vertical lines drawn during the first step. In some embodiments, vertical lines may be drawn corresponding to the lateral edges of the pedicles instead of lines corresponding to the midpoints of the pedicles.

The oblique view lines may be about 2 cm to about 3 cm away from the lateral pedicle border lines marked in the first step. For larger patients, the oblique view line may be greater than about 3 cm away from the midlife marked in the first step. For smaller patients, the oblique view line may be closer than about 2 cm away from the midline marked in the first step. The intersection of the oblique view lines with the horizontal lines drawn in the second step may represent skin entry points for a targeting needle as the targeting needle passes through soft tissue at an angle towards the bony pedicle entry point. A side fluoroscopic image, the horizontal lines, and the vertical lines may help the surgeon triangulate between the skin entry points and bony entry points.

Fourth, an incision may be made in the skin between mid-pedicle lines along the vertical oblique view lines. The skin incision may be from about 2 cm to about 4 cm long. The incision may be from about 2.5 cm to about 3 cm long. Limiting the length of the incision may enhance patient satisfaction with the procedure. The incisions may be pre-anesthetized with, for example, 1% lidocaine with 1:200,000 epinephrine. To blunt the pain response, a long spinal needle may be used to dock on the bone entry point and inject the planned muscle path in a retrograde fashion as well. Once the incision has been made, tissue surrounding the incision may be pulled and/or stretched to allow access to a target location in a vertebra.

After sterile preparation and draping, the pedicle entry points may be fluoroscopically rechecked to ensure that the previously marked lines correspond to the intersection of the midline of the transverse process and the lateral joint and pars interarticularis. The intersection of the facet and the transverse process provides a starting point that may help avoid the canal and follow the natural inclination of lumbar pedicles. For the spinal stabilization system described, in which sleeves coupled to bone fastener assemblies are substantially unconstrained by insertion angles of the bone fasteners, patient anatomy may determine the most advantageous insertion angles of the bone fasteners.

A scalpel may be used to make a stab wound at the junction of an oblique view line and a mid-pedicle line. The scalpel may be a #11 scalpel. A targeting needle may be passed through the incision in an oblique lateral to medial trajectory towards the bony entry point defined by a lateral pedicle border line. The C-arm of the fluoroscope may be placed in an anteroposterior position for this maneuver.

As the targeting needle encounters the bony anatomy, anteroposterior fluoroscopic images may be used to place the tip of the needle at the upper outer quadrant of the pedicle. The needle may be walked medially along the transverse process to the pedicle entry point. The needle tip may be docked by lightly tapping the tip into the bone with a mallet or other impact device to drive the tip into the bone. In some embodiments, the needle tip may be docked by applying downward pressure to the targeting needle to force the tip into the bone.

The fluoroscope may then be moved to a lateral position. The surgeon may correct the sagittal trajectory of the needle by moving the needle in an anterior or posterior direction to match the vector of the pedicle corridor. A mallet or other impact device may be used to gently advance the targeting needle into the pedicle halfway to the pedicle-vertebral body junction. Force may be applied to the targeting needle to drive the targeting needle into the pedicle halfway to the pedicle-vertebral body junction. An anteroposterior image may then be obtained to confirm that the needle is approximately halfway across the pedicle in the anteroposterior view. If the tip is more than halfway across the pedicle in a lateral to medial projection, the trajectory may be too medial. Further advancement of the needle may risk passing the needle through the spinal canal. The needle may be repositioned. A new starting point or new trajectory may be obtained. If the anteroposterior image demonstrates that the needle is significantly lateral in the pedicle, then the needle may have passed along the lateral portion of the pedicle. A needle that has passed along the lateral portion of the pedicle may be withdrawn and repositioned.

Once a good trajectory has been obtained, the targeting needle may be advanced using a mallet. The needle may be pushed in without a mallet. The targeting needle may be advanced to the junction of the pedicle and vertebral body under lateral fluoroscopic guidance.

After targeting needle has been advanced into the bone, a guide wire may be placed through a passage in targeting needle into a vertebral body. Once the guide wire has been passed through the targeting needle and the targeting needle has been removed, the guide wire may be used as a guide to position one or more successively sized dilators around a target location in a pedicle. A dilator may be a conduit with a regular shape (e.g., cylindrical) or an irregular shape (e.g., C-shaped). A dilator may form an opening through soft tissue to the pedicle. For patients with a thick fascia, it may be advantageous to make a nick in the fascia with a scalpel blade to facilitate passage of the dilators. The dilators may be passed sequentially over the guide wire. The dilators may be rotated during insertion to facilitate dilation of surrounding tissue. The dilators may be inserted until the leading edges contact the pedicle. A distal end of a dilator may be tapered to facilitate positioning of the dilator proximate the pedicle. An instrumentation set for a spinal stabilization system may include two, three, four, or more successively sized dilators.

After tissue dilation has been achieved, a large diameter dilator may be used to guide a bone fastener assembly and/or insertion instruments toward a target location in a pedicle.

A bone fastener assembly with a bone fastener of an appropriate length may be selected for insertion in a patient. The size of the bone fastener may be verified with measurement indicia in an instrumentation set. Measurement indicia may be etched or printed on a portion of an instrumentation set. For example, the chosen bone fastener embodiment may be placed over the outline of a bone fastener embodiment printed on a tray of the instrumentation set.

The chosen bone fastener assembly may be attached to a detachable member. A bone fastener assembly may be rotated on a flange of a detachable member. Movable members of the detachable member may be extended into indentations in a collar of the bone fastener assembly. A driver may be used to extend the movable members to couple with the collar. When the bone fastener assembly is coupled to the detachable member, a drive portion of a fastener driver may be coupled to a tool portion of the bone fastener. A shaft of the fastener driver may be positioned in the passage of the detachable member. A removable handle may be attached to the shaft of the fastener driver. The detachable member, collar, and bone fastener may be substantially co-axial when the fastener driver is positioned in the detachable member. The removable handle may be attached to the shaft of the fastener driver after the bone fastener, collar, detachable member, and fastener driver combination is positioned down a guide wire through a dilator and against a pedicle.

After the bone fastener has been secured to the vertebra and the driver has been removed from the sleeve, the polyaxial nature of the collar may allow angulation of the sleeve relative to the bone fastener. Tissue surrounding the incision may be released such that the sleeve is angled toward a central location between vertebrae to be stabilized. The sleeve may be moved to facilitate positioning of instruments and/or to facilitate access to the adjacent vertebra that is to be stabilized. For example, the sleeve may be tilted towards the adjacent pedicle so that additional length of an opening in the patient is not needed. The channel in the sleeve may be turned toward the adjacent pedicle that is to be stabilized with the spinal stabilization system being formed.

A plane of dilated tissue may be created between a first pedicle and a second pedicle to be stabilized with a spinal stabilization system. A bone fastener assembly and a sleeve may be coupled to the first pedicle. The second pedicle may be adjacent to the first pedicle. In an embodiment, a tissue wedge may be placed in the sleeve coupled to the first pedicle such that the distal end of the tissue wedge contacts the head of the bone fastener. The proximal end of the sleeve coupled to the first pedicle may be held such that tissue around the incision is not pulled or stretched. The tissue wedge may be moved back and forth (wanded) through the channel in the sleeve and the slot in the collar toward the target location at the second pedicle, thereby creating a plane in muscle and other tissue between the head of the installed bone fastener and the target location of a second bone fastener. A tissue wedge may be pivoted about an inside proximal edge of the sleeve such that the distal end of the tissue wedge bluntly splits the muscle and fascia along fibers and create a tissue plane between the two pedicles. The back and forth (wanding) action may be repeated more than once (e.g., two or three times) to create a good working plane and displace unwanted tissue from the plane. The wanding may create a tissue plane. The tissue plane may be substantially trapezoidal. A tissue plane may be created before a bone fastener assembly is inserted into a vertebra.

A tissue plane may be made in a variety of shapes including, but not limited to, substantially trapezoidal, substantially rhomboidal, and substantially triangular. A tissue plane with a substantially geometric shape may have the basic geometric shape with, for example, slightly curved edges and/or slightly rounded corners or apices. A sleeve length may be chosen to reduce a size of a tissue plane that needs to be formed between pedicles. Creating a trapezoidal tissue plane may reduce the invasiveness of a procedure. Limiting the area of the plane may promote a faster recovery time and/or may reduce an amount of post-operative pain experienced by the patient.

Two pedicles may be targeted and bone fastener assemblies anchored in both pedicles before creation of a tissue plane. A tissue wedge may be inserted at either of the pedicles. The sleeves may be coupled to each other at proximal ends of the sleeves. The tissue wedge may be coupled to a sleeve and the sleeve may be used as an anchor during wanding. Insertion of a rod into collars of bone fastener assemblies, however, may require cutting of some tissue between the two sleeves.

Other procedures may be used to create a tissue plane. For example, before targeting pedicle locations (i.e., before bone fastener insertion), a tissue wedge may be worked downward from an incision to create a tissue plane. Alternatively, a scalpel may be used to cut from the surface of the body to vertebral bone. Extensive use of a scalpel, however, may remove benefits of a minimally invasive procedure.

With bone fastener assemblies secured in the vertebral bodies, sleeves coupled to the bone fastener assemblies may be oriented to facilitate insertion of a rod in the sleeves. Sleeves may serve as tissue retractors during a spinal stabilization procedure. Angular motion of a collar may be limited by a range of motion allowed between the collar and the bone fastener that the collar is anchored to. Angular motion of a collar may be limited by patient anatomy. Angular motion or orientation of one collar (i.e., sleeve),however, may not depend upon a position of another collar (i.e., sleeve). Channel openings in the sleeves may face each other. Channel openings in the sleeves may be angled relative to each other in various arrangements. A distance between the sleeves may be estimated using an estimating tool. The distance between the sleeves may be used to select a length of a rod needed to couple the collars.

A rod or a portion of a rod may be cut to length and contoured as desired. For example, a medical practitioner may use experience and judgment to determine curvature of a rod for a patient. A desired curvature for the rod may be determined using fluoroscopic imaging. In some embodiments, a curvature of the rod may be chosen such that, when the rigid portion 104B of rod 104 is secured to collars 112 of bone fastener assemblies 102, sleeves coupled to bone fastener assemblies 102 cross at a surface of the skin. Crossing of the sleeves at a surface of the skin allows the medical practitioner to minimize trauma to a patient by minimizing incision length and tissue plane area. The rod may be bent or shaped with a tool (e.g., a rod bender) to allow insertion of the rod through channels of sleeves with various spatial locations and/or various angular orientations. In some embodiments, dynamic portion 104A of rod 104 may be cut to length. In some embodiments, rigid portion 104B of rod 104 may be cut to length. In some embodiments, both dynamic portion 104A and rigid portion 1048 may be cut to length.

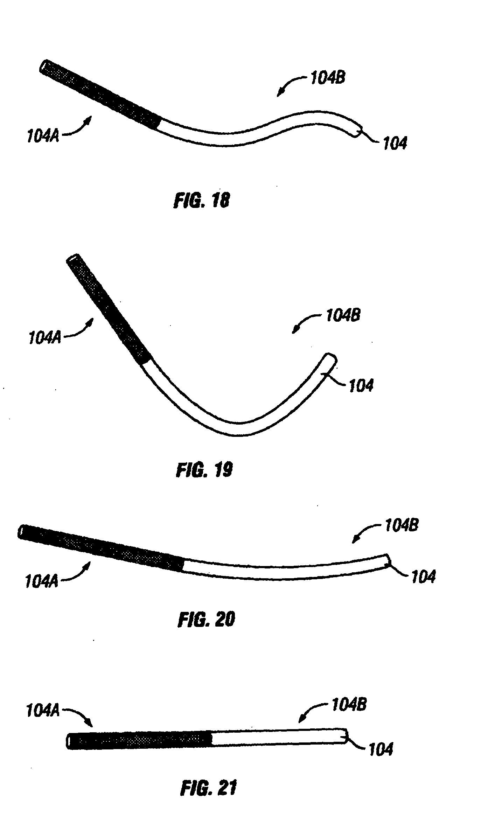

In some embodiments, rigid portion 104B of rods 104 may have shapes including, but not limited to, straight, bent, curved, s-shaped, and z-shaped. FIGURE 18 depicts an embodiment of rod 104 with rigid portion 104B having an S-shape. FIGURE 19 depicts an embodiment of rod 104 with rigid portion 104B having an angled shape. FIGURE 20 depicts an embodiment of rod 104 with rigid portion 104B having a bent shape. FIGURE 21 depicts an embodiment of rod 104 with rigid portion 104B having a straight shape. In some embodiments, rods 104 may have a substantially circular longitudinal cross section. An instrumentation kit for a spinal stabilization system may include straight rods and/or pre-shaped rods. Straight rods and/or pre-shaped rods may be contoured to accommodate patient anatomy if needed during the surgical procedure.

Channels of the sleeves and slots of the collars may be oriented by rotating the sleeves to accommodate insertion and seating of the rod. A channel opening in a sleeve may be non-linear (e.g., bent, curved, or angled) to allow portions of the spine to be selectively stabilized. Sleeve orientation and/or design may be chosen to allow compression, distraction, and/or reduction of vertebrae. There may be no constraints governing relative location and/or orientation of the sleeves. Sleeves may be forced apart or angled toward each other or away from each other to accommodate insertion of the rod.

Prior to insertion of the rod, the tissue wedge or targeting needle may be used to wand between the bone fasteners to ensure a clean plane between the bone fasteners. An end of the rod may be inserted at an angle or substantially longitudinally in a passage and/or channel of a sleeve coupled to a bone fastener assembly. Inserting the rod at an angle or substantially longitudinally allows the length of the incision and/or the area of the tissue plane to remain advantageously small. Sleeves coupled to anchored bone fastener assemblies may remain essentially unconstrained relative to each other during insertion of the rod. In certain embodiments, angular orientation of the collars may determine a trajectory of the rod down the sleeves and into collars of the bone fastener assemblies. Inserting the rod down two or more sleeves and through an open path (i.e., the tissue plane) may allow a medical practitioner to avoid surgical difficulties associated with anatomical abnormalities and/or misalignment of system components.

Insertion of rod 104 may not be visualized subcutaneously. Therefore, a positioning tool may be used to guide rod 104 down the sleeves into slots in collars 112 and may further position rod 104 in blocking body 105. A distal portion of the positioning tool may be contoured. The contour may allow for some rotation of rod 104. With slight pressure, rod 104 may be rotated subcutaneously into a substantially horizontal position and seated in collars 112. The positioning tool may be held firmly while still allowing a rocking movement between the rod and the distal end of the positioning tool. Movement of the rod may allow the rod to be maneuvered down the sleeves and into the collars. Blocking body 105 may be coupled to rod 104 and advanced with rod 104 via sleeves. Ligature 14, blocking body 105 and rod 104 may be positioned in the patient before bone fastener assemblies 102. For example, ligature 14 may be passed around a non-pedicle portion of the third vertebra, rod 104 may be advanced into the patient, and blocking body 105 may couple rod 104 to third vertebra using ligature 14, before bone fastener assemblies 102 are positioned in the body.

After rod 104 is seated in collars 112, additional fluoroscopic confirmation of rod positioning may be obtained, which may include confirming the positioning of dynamic portion 104A in blocking body 105 and rigid portion 104B in collars 112. With rod 104 satisfactorily positioned, rod 104 may be secured in place by tensioning ligature 14 to a non-pedicle portion of the vertebra and advancing closure members 106 in blocking body 105 and collars 112. To ensure alignment of thread of closure member 106 with thread of collar 112 or blocking body 105, the driver may initially be rotated in a direction that would result in removal of closure member 106 from collar 112 or blocking body 105. When the user of the driver feels engagement of threading of the closure member with threading, the user may reverse the direction of rotation of the driver to secure the closure member. Closure member 106 may secure rod 104 to collars 112 and blocking body 105. A sleeve may serve as a coaxial guide to inhibit cross-threading during insertion of closure members 106. When closure members 106 are seated and rod 104 is secured, collars 112 are angled such that slots in the collars are substantially perpendicular to the rod. A driver may be disengaged from the closure member and removed from the sleeve. Adjacent Level Disease (ALD) may be prevented or slowed down in the patient by rod 104 having a dynamic portion 104A allowing movement of the third vertebra relative to the first and second vertebrae while the rigid portion 104B fuses the first and second vertebrae.

A counter torque wrench may be used to inhibit application of torque to a patient's spine. Force may be applied to the counter torque wrench in a direction opposite to rotational force applied to a driver.

A spinal stabilization system may be inserted using an invasive procedure. Since insertion of a spinal stabilization system in an invasive procedure may be visualized, cannulated components (e.g., bone fasteners) and/or instruments (e.g., detachable members) may not be needed for the invasive (i.e., open) procedure. Thus, a bone fastener used in an invasive procedure may differ from a bone fastener used in a minimally invasive procedure.

Tools used in an invasive procedure may be similar to tools used in a minimally invasive procedure. Methods of installing a spinal stabilization system in an invasive procedure may be similar to methods of installing a spinal stabilization system in a minimally invasive procedure.

## Claims

1. A spinal stabilization system (100) having a first anchor for anchoring the spinal stabilization system into a pedicle of a first vertebra and a second anchor for anchoring the spinal stabilization system into a pedicle of a second vertebra, further comprising:
a rod (104);
a conformable ligature (14) comprising a first end and a second end and a loop portion for passing around a portion of an adjacent third vertebra; and
a blocking body (105) connecting the conformable ligature (14) and a portion of the rod (104), the blocking body (105) comprising:
a first longitudinal element (20) having an inside face (20d), an outside face (20c), and a loop passage (54) extending between the inside face (20d) and the outside face (20c) of the first longitudinal element (20);
a second longitudinal element (22) having an inside face (22d), an outside face (22c), and an exit passage (40) extending between the inside face (22d) and the outside face (22c) of the second longitudinal element (22), wherein the loop portion of the conformable ligature (14) passes through the loop passage (54) and the first and second ends of the conformable ligature (14) extend from the exit passage (40), and wherein the inside faces (20d, 22d) of the first and second longitudinal elements (20, 22) have substantially semi-cylindrically shaped recesses for receiving the portion of the rod (104) together with a portion of the conformable ligature (14); and
a closure member (106) for securely coupling the first and second longitudinal elements (20, 22) of the blocking body (105) to immobilize the blocking body (105) relative to the portion of the rod (104) and to fix in position the portion of the conformable ligature (14) relative to the substantially semi-cylindrically shaped recesses of the first and second longitudinal elements (20, 22),
**characterized in that**
the rod (104) has a rigid portion (104B) and a dynamic portion (104A), wherein the dynamic portion has an interlocking pattern, wherein the portion of the rod (104) received by the recesses and relative to which the blocking body (105) is immobilized is the dynamic portion (104A).

2. The system of claim 1, wherein the rod comprises:
a cylindrical body, wherein the dynamic portion comprises:
a cannulated interior and an opening extending spirally around a longitudinal axis of the cylindrical body; and
a biomaterial coating the cylindrical body in whole or in part and at least partially filling the opening.

3. The system of claim 2, wherein the opening forms the interlocking pattern.

4. The system of claim 3, wherein the interlocking pattern has a shape of a dog bone.

5. The system of claim 2, wherein the interlocking pattern has a shape of a puzzle.

6. The system of claim 2, wherein the biomaterial fills the cylindrical body in whole or in part.

7. The system of claim 2, wherein the biomaterial is polycarbonate urethane.

## Patentansprüche

1. Wirbelsäulenstabilisierungssystem (100) mit einem ersten Anker zum Verankern des Wirbelsäulenstabilisierungssystems in einen Pedikel eines ersten Wirbels und einem zweiten Anker zum Verankern des Wirbelsäulenstabilisierungssystems in einen Pedikel eines zweiten Wirbels, ferner umfassend:
einen Stab (104);
ein anpassbares Band (14), das ein erstes Ende und ein zweites Ende und einen Schleifenabschnitt zur Führung um einen Abschnitt eines benachbarten dritten Wirbels herum umfasst; und
einen Blockierkörper (105), der das anpassbare Band (14) und einen Abschnitt des Stabs (104) verbindet, wobei der Blockierkörper (105) umfasst:
ein erstes Längselement (20) mit einer Innenseite (20d), einer Außenseite (20c) und einem Schleifendurchgang (54), der sich zwischen der Innenseite (20d) und der Außenseite (20c) des ersten Längselements (20) erstreckt;
ein zweites Längselement (22) mit einer Innenseite (22d), einer Außenseite (22c) und einem Austrittsdurchgang (40), der sich zwischen der Innenseite (22d) und der Außenseite (22c) des zweiten Längselements (22) erstreckt, wobei der Schleifenabschnitt des anpassbaren Bands (14) durch den Schleifendurchgang (54) führt und sich das erste und zweite Ende des anpassbaren Bands (14) von dem Austrittsdurchgang (40) erstrecken, und wobei die Innenseiten (20d, 22d) des ersten und zweiten Längselements (20, 22) im Wesentlichen halbzylinderförmige Aussparungen zum Aufnehmen des Abschnitts des Stabs (104) zusammen mit einem Abschnitt des anpassbaren Bands (14) aufweisen; und
ein Schließelement (106) zum festen Koppeln des ersten und zweiten Längselements (20, 22) des Blockierkörpers (105), um den Blockierkörper (105) relativ zu dem Abschnitt des Stabs (104) zu immobilisieren und um den Abschnitt des anpassbaren Bands (14) relativ zu den im Wesentlichen halbzylinderförmigen Aussparungen des ersten und zweiten Längselements (20, 22) an seiner Stelle zu befestigen,
**dadurch gekennzeichnet, dass**
der Stab (104) einen starren Abschnitt (104B) und einen dynamischen Abschnitt (104A) aufweist, wobei der dynamische Abschnitt ein ineinandergreifendes Muster aufweist, wobei der Abschnitt des Stabs (104), der durch die Aussparungen aufgenommen wird und relativ zu dem der Blockierkörper (105) immobilisiert wird, der dynamische Abschnitt (104A) ist.

2. System nach Anspruch 1, wobei der Stab umfasst:
einen zylinderförmigen Körper, wobei der dynamische Abschnitt umfasst:
eine Innenseite mit Kanal und eine Öffnung, die sich spiralförmig um eine Längsachse des zylinderförmigen Körpers herum erstreckt; und
ein Biomaterial, das den zylinderförmigen Körper vollständig oder teilweise beschichtet und die Öffnung zumindest teilweise füllt.

3. System nach Anspruch 2,
wobei die Öffnung das ineinandergreifende Muster bildet.

4. System nach Anspruch 3,
wobei das ineinandergreifende Muster hundeknochenförmig ist.

5. System nach Anspruch 2,
wobei das ineinandergreifende Muster die Form eines Puzzles aufweist.

6. System nach Anspruch 2,
wobei das Biomaterial den zylinderförmigen Körper vollständig oder teilweise füllt.

7. System nach Anspruch 2,
wobei das Biomaterial Polycarbonaturethan umfasst.

## Revendications

1. Système de stabilisation spinale (100) ayant un premier ancrage pour ancrer le système de stabilisation spinale dans un pédicule d'une première vertèbre et un second ancrage pour ancrer le système de stabilisation spinale dans un pédicule d'une seconde vertèbre, comprenant en outre :
une tige (104) ;
une ligature (14) susceptible d'être conformée, comprenant une première extrémité et une seconde extrémité et une portion en boucle pour passer autour d'une portion d'une troisième vertèbre adjacente ; et
un corps de blocage (105) qui connecte la ligature (14) susceptible d'être conformée et une portion de la tige (104), le corps de blocage (105) comprenant :
un premier élément longitudinal (20) ayant une face interne (20d), une face externe (20c), et un passage en boucle (54) s'étendant entre la face interne (20d) et la face externe (20c) du premier élément longitudinal (20) ;
un second élément longitudinal (22) ayant une face interne (22d), une face externe (22c) et un passage de sortie (40) s'étendant entre la face interne (22d) et la face externe (22c) du second élément longitudinal (22), dans lequel la portion en boucle de la ligature (14) susceptible d'être conformée passe à travers le passage en boucle (54), et la première et la seconde extrémité de la ligature (14) susceptible d'être conformée s'étendent depuis le passage de sortie (40), et dans lequel les faces internes (20d, 22d) des premier et second éléments longitudinaux (20, 22) ont des évidements de forme sensiblement semi-cylindrique pour recevoir la portion de la tige (104) ensemble avec une portion de la ligature (14) susceptible d'être conformée ; et
un élément de fermeture (106) pour coupler de façon assurée les premier et second éléments longitudinaux (20, 22) du corps de blocage (105) pour immobiliser le corps de blocage (105) par rapport à la portion de la tige (104) et pour fixer en position la portion de la ligature (14) susceptible d'être conformée par rapport aux évidements de forme sensiblement semi-cylindrique des premier et second éléments longitudinaux (20, 22),
**caractérisé en ce que**
la tige (104) a une portion rigide (104B) et une portion dynamique (104A), telles que la portion dynamique possède un motif d'interverrouillage, dans lequel la portion de la tige (104) reçue par les évidements et par rapport à laquelle le corps de blocage (105) est immobilisé est la portion dynamique (104A).

2. Système selon la revendication 1, dans lequel la tige comprend :
un corps cylindrique, et la portion dynamique comprend :
un intérieur en canule et une ouverture s'étendant en spirale autour d'un axe longitudinal du corps cylindrique ; et
un biomatériau qui revêt le corps cylindrique en totalité ou en partie et qui remplit au moins partiellement l'ouverture.

3. Système selon la revendication 2, dans lequel l'ouverture forme le motif d'interverrouillage.

4. Système selon la revendication 3, dans lequel le motif d'interverrouillage présente une forme en haltère.

5. Système selon la revendication 2, dans lequel le motif d'interverrouillage présente une forme en puzzle.

6. Système selon la revendication 2, dans lequel le biomatériau remplit le corps cylindrique en totalité ou en partie.

7. Système selon la revendication 2, dans lequel le biomatériau est du polycarbonate uréthane.
